(19) Europäisches Patentamt
European Patent Office
Office européen des brevets

(11) EP 4 501 630 A1

(12) **EUROPEAN PATENT APPLICATION**
published in accordance with Art. 153(4) EPC

(43) Date of publication:
05.02.2025 Bulletin 2025/06

(21) Application number: 23781102.1

(22) Date of filing: 31.03.2023

(51) International Patent Classification (IPC):
B32B 27/00 (2006.01)     A61K 9/70 (2006.01)
A61F 13/02 (2024.01)     C09J 7/38 (2018.01)
B32B 7/06 (2019.01)

(52) Cooperative Patent Classification (CPC):
A61F 13/02; A61K 9/70; B32B 7/06; B32B 27/00;
C09J 7/38

(86) International application number:
PCT/JP2023/013695

(87) International publication number:
WO 2023/191094 (05.10.2023 Gazette 2023/40)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 31.03.2022 JP 2022059979
30.06.2022 JP 2022106223

(71) Applicant: Toppan Holdings Inc.
Tokyo 110-0016 (JP)

(72) Inventors:
• NOMURA, Saeko
Tokyo 110-0016 (JP)
• TANABE, Junya
Tokyo 110-0016 (JP)
• MIYAUCHI, Natsumi
Tokyo 110-0016 (JP)

(74) Representative: TBK
Bavariaring 4-6
80336 München (DE)

(54) **AFFIXING BODY, AND METHOD FOR MANUFACTURING AFFIXING BODY**

(57) An adhesive tape includes a substrate that is in contact with a carrier sheet and has a higher tensile elongation at break than the carrier sheet and a release sheet, and an adhesive layer that is in contact with the release sheet and is configured to be adhered to a living body surface after the release sheet is peeled off from the adhesive layer. The carrier sheet has a larger area than the adhesive tape, which is located within the carrier sheet in a view facing a plane where the carrier sheet extends. The carrier sheet includes a slit extending toward the adhesive tape from a portion of an outer edge of the carrier sheet that does not overlap the adhesive tape and having a length that does not reach an outer edge of the adhesive tape in the view facing the plane where the carrier sheet extends.

Fig.1

EP 4 501 630 A1

**Description**

TECHNICAL FIELD

The present disclosure relates to an adhesive body and a method for manufacturing an adhesive body.

BACKGROUND

Adhesive bodies for the skin are widely used with the objectives of protecting affected areas on the skin from external influences or concealing parts of the skin from external view. The adhesive body includes an adhesive tape located between a carrier sheet and a release sheet. The adhesive tape includes a substrate layer that is in contact with the carrier sheet and an adhesive layer that is in contact with the release sheet. To use the adhesive body, the release sheet is first peeled off from the adhesive layer. Then, the laminate of the adhesive tape and the carrier sheet is adhered to the skin with the adhesive layer. Subsequently, the carrier sheet is peeled off from the substrate layer (for example, see Patent Literature 1).

CITATION LIST

Patent Literature

Patent Literature 1: Japanese Patent No. 6706398

SUMMARY OF INVENTION

Technical Problem

The substrate layer to be adhered to the skin is required to have a relatively high tensile elongation at break, like polyurethane, to achieve conformity with the skin. However, such a substrate layer also has a relatively high adhesion to carrier sheets made of polyolefin or polyester, making it difficult to peel off the carrier sheet from the substrate layer adhered to the skin. This issue arises not only when the adhesive tape is applied to human skin but also when it is used on the body surfaces of non-human animals, such as the body surfaces of mammals.

Solution to Problem

An aspect of an adhesive body includes a carrier sheet, a release sheet, and an adhesive tape located between the carrier sheet and the release sheet. The adhesive tape includes a substrate that is in contact with the carrier sheet and has a higher tensile elongation at break than the carrier sheet and the release sheet and an adhesive layer that is in contact with the release sheet and is configured to be adhered to a living body surface. The carrier sheet has a larger area than the adhesive tape, and the adhesive tape is located within the carrier sheet as seen in a view facing a plane on which the carrier sheet extends. The carrier sheet includes a slit extending toward the adhesive tape from a portion of an outer edge of the carrier sheet that does not overlap the adhesive tape and having a length that does not reach an outer edge of the adhesive tape as seen in the view facing the plane on which the carrier sheet extends.

An aspect of an adhesive body includes a carrier sheet, a release sheet, and an adhesive tape located between the carrier sheet and the release sheet. The adhesive tape includes a substrate that is in contact with the carrier sheet and has a higher tensile elongation at break than the carrier sheet and the release sheet, and includes an adhesive layer that is in contact with the release sheet. The adhesive layer is configured to be adhered to a living body surface after the release sheet is peeled off from the adhesive layer. The carrier sheet has a larger area than the adhesive tape, and the adhesive tape is located within the carrier sheet as seen in a view facing a plane on which the carrier sheet extends. The carrier sheet includes a slit extending toward the adhesive tape from a portion of an outer edge of the carrier sheet that does not overlap the adhesive tape and having a length that does not reach an outer edge of the adhesive tape as seen in the view facing the plane on which the carrier sheet extends. In a tear test using a right angled tear method in accordance with JIS K 7128 3:1998, a displacement amount (mm) of the adhesive tape at break is 3.0 times or more than a displacement amount (mm) of the carrier sheet at break. An integral value of a tear load applied to the carrier sheet until the carrier sheet breaks is less than or equal to 40 N·mm.

An aspect of an adhesive body includes a release layer, a carrier layer, an adhesive layer located between the release layer and the carrier layer, the adhesive layer having an adhesive surface that is in contact with the release layer, and a substrate layer that is located between the adhesive layer and the carrier layer, is in contact with the adhesive layer and the carrier layer, and has a higher tensile elongation at break than the release layer and the carrier layer. In the adhesive body, the adhesive surface from which the release layer has been peeled off is configured to be adhered to skin. The adhesive body further includes a gap in which the substrate layer is separated from the carrier layer. The gap is located in at least part of an outer shape of a laminate that includes the adhesive layer and the substrate layer. An adhesion strength between the substrate layer and the carrier layer is less than or equal to 2 N/25 mm.

An aspect of a method for manufacturing an adhesive body includes forming a precursor such that an adhesive layer having an adhesive surface that is in contact with a release layer and is located between the release layer and a carrier layer and such that a substrate layer that is in contact with the adhesive layer and the carrier layer and is located between the adhesive layer and the carrier layer, the substrate layer having a higher tensile elongation at break than the release layer and the carrier layer, and forming a gap in which the substrate layer is separated from the carrier layer, the gap being located in at least part of an outer shape of a laminate that includes the substrate layer and the carrier layer. An adhesion strength between the substrate layer and the carrier layer is less than or equal to 2 N/25 mm.

BRIEF DESCRIPTION OF DRAWINGS

Fig. 1 is a cross-sectional view showing the structure of an adhesive body according to an embodiment.
Fig. 2 is a plan view showing the structure of the adhesive body shown in Fig. 1.
Fig. 3 is a plan view showing the structure of the adhesive body having two slits.
Fig. 4 is a plan view showing the structure of the adhesive body having two slits.
Fig. 5 is a plan view showing the structure of the adhesive body having two slits.
Fig. 6 is a plan view showing the structure of the adhesive body having two slits.
Fig. 7 is a plan view showing the structure of the adhesive body having two slits.
Fig. 8 is a plan view showing the structure of the adhesive body having two slits.
Fig. 9 is a plan view showing the structure of the adhesive body including a circular adhesive tape.
Fig. 10 is a plan view showing the structure of an adhesive body including a first another example of the adhesive tape.
Fig. 11 is a plan view showing the structure of an adhesive body including a second another example of the adhesive tape.
Fig. 12 is a process diagram illustrating an adhering step for the adhesive tape shown in Fig. 11.
Fig. 13 is a process diagram illustrating the adhering step for the adhesive tape shown in Fig. 11.
Fig. **14** is a process diagram illustrating the adhering step for the adhesive tape shown in Fig. 11.
Fig. 15 is a cross-sectional view showing the structure of a first example of the adhesive body including adhesive tapes.
Fig. 16 is a plan view showing the structure of the adhesive body shown in Fig. 15.
Fig. 17 is a cross-sectional view showing the structure of a second example of the adhesive body including adhesive tapes.
Fig. 18 is a cross-sectional view showing the structure of a third example of the adhesive body including adhesive tapes.
Fig. 19 is a cross-sectional view showing the structure of a fourth example of the adhesive body including adhesive tapes.
Fig. 20 is a cross-sectional view showing the structure of the adhesive body according to a second embodiment.
Fig. 21 is a plan view showing the structure of the adhesive body shown in Fig. 20.
Fig. 22 is a plan view showing the structure of a test piece.
Fig. 23 is a graph illustrating the relationship between the displacement amount and tear resistance of the adhesive tape.
Fig. **24** is a graph illustrating the relationship between the displacement amount and tear resistance of the adhesive tape.
Fig. 25 is a graph illustrating the relationship between the displacement amount and tear resistance of the carrier sheet.
Fig. 26 is a graph illustrating the relationship between the displacement amount and tear load of the carrier sheet.
Fig. 27 is a plan view showing the structure of a first example of the adhesive body according to a third embodiment.
Fig. 28 is a cross-sectional view showing the structure taken along line XXVI-XXVI shown in Fig. 25.
Fig. 29 is a plan view showing the structure of a second example of the adhesive body in a third embodiment.
Fig. 30 is a cross-sectional view showing the structure taken along line XXVIII-XXVIII shown in Fig. 27.
Fig. 31 is a process diagram for illustrating a method for manufacturing the adhesive body in the third embodiment.
Fig. 32 is a process diagram for illustrating the method for manufacturing the adhesive body in the third embodiment.
Fig. 33 is a process diagram for illustrating the method for manufacturing the adhesive body in the third embodiment.
Fig. 34 is a process diagram for illustrating the method for manufacturing the adhesive body in the third embodiment.

Fig. 35 is a process diagram for illustrating the method for manufacturing the adhesive body in the third embodiment.

Fig. 36 is a plan view showing the structure of the adhesive body according to a fourth embodiment.

Fig. 37 is an enlarged plan view showing part of the adhesive body shown in Fig. 36.

Fig. 38 is a process diagram for illustrating the method for manufacturing the adhesive body in the fourth embodiment.

Fig. 39 is a process diagram for illustrating the method for manufacturing the adhesive body in the fourth embodiment.

MODES FOR CARRYING OUT THE INVENTION

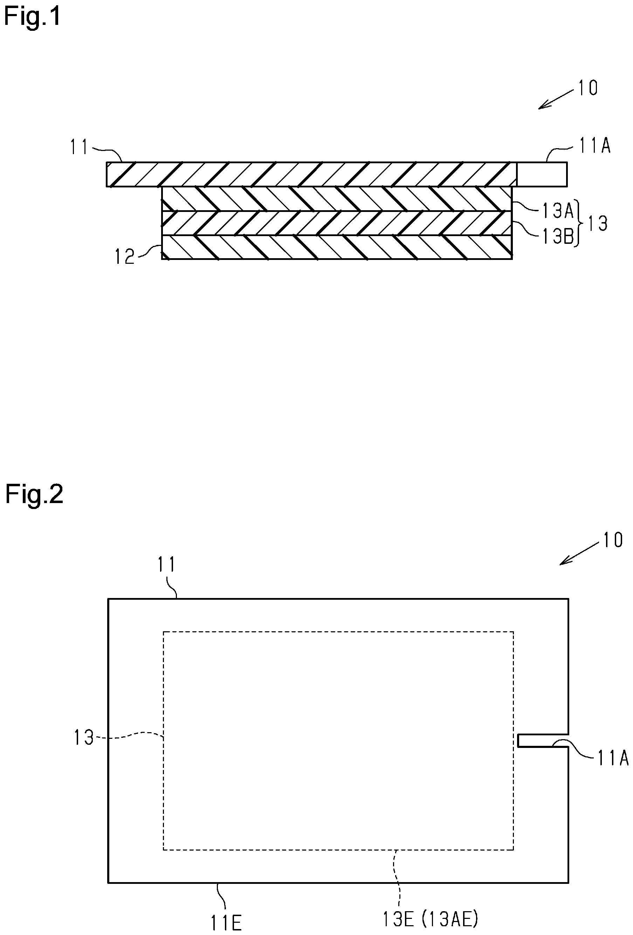

An adhesive body according to a first embodiment will now be described with reference to Figs. 1 and 2.

Structure

Fig. 1 shows the structure of the adhesive body in a cross-section that is orthogonal to the plane in which the adhesive body extends.

As shown in Fig. 1, the adhesive body 10 includes a carrier sheet 11, a release sheet 12, and an adhesive tape 13. The adhesive tape 13 is located between the carrier sheet 11 and the release sheet 12. The adhesive tape 13 includes a substrate 13A and an adhesive layer 13B. The substrate 13A is in contact with the carrier sheet 11. The substrate 13A has a higher tensile elongation at break than the carrier sheet 11 and the release sheet 12. The adhesive layer 13B is in contact with the release sheet 12. The adhesive layer 13B is adhered to a living body surface after the release sheet 12 is peeled off from the adhesive layer 13B. In the present embodiment, the adhesive layer 13B is laminated on the substrate 13A.

The living body surface to which the adhesive layer 13B is adhered is, for example, human skin, but may be body surfaces of animals other than humans, for example, mammals other than humans. In the present embodiment, an exemplary case in which the adhesive layer 13B is adhered to human skin will be described.

Fig. 2 shows a structure of the adhesive body 10 as seen in a view facing a plane on which the carrier sheet 11 extends.

As shown in Fig. 2, the area of the carrier sheet 11 is larger than the area of the adhesive tape 13. The adhesive tape 13 is located within the carrier sheet 11 as seen in the view facing the plane on which the carrier sheet 11 extends. In the present embodiment, the area of the carrier sheet 11 is larger than the area of the adhesive tape 13 and the area of the release sheet 12. In the adhesive tape 13, the size of the substrate 13A is equal to the size of the adhesive layer 13B. That is, the area of the substrate 13A is equal to the area of the adhesive layer 13B, and the shape of the substrate 13A is the same as the shape of the adhesive layer 13B.

The size of the adhesive tape 13 is equal to the size of the release sheet 12. That is, the area of the adhesive tape 13 is equal to the area of the release sheet 12, and the shape of the adhesive tape 13 is the same as the shape of the release sheet 12. The area of the release sheet 12 may be larger than the area of the adhesive tape 13, and the adhesive tape 13 may be located within the release sheet 12. That is, the size of the release sheet 12 may be greater than or equal to the size of the adhesive tape 13.

The carrier sheet 11 includes a slit 11A that extends toward the adhesive tape 13 from a portion of an outer edge 11E of the carrier sheet 11 that does not overlap the adhesive tape 13 and has a length that does not reach an outer edge 13E of the adhesive tape 13 as seen in the view facing the plane on which the carrier sheet 11 extends. That is, the slit 11A of the carrier sheet 11 is shaped to extend from the outer edge 11E toward the inside of the outer edge 11E. The slit 11A extends through the carrier sheet 11 in the thickness direction of the carrier sheet 11. In the present embodiment, the slit 11A is a straight I-notch.

In the slit 11A, the end of the slit 11A at the outer edge 11E is a basal end, and the end opposite to the basal end is a distal end. The distal end of the slit 11A is located between the outer edge 11E of the carrier sheet 11 and an outer edge 13AE of the substrate 13A as seen in the view facing the plane on which the carrier sheet 11 extends. Thus, since the slit 11A has a length that does not reach the outer edge 13AE of the substrate 13A of the adhesive tape 13, the entire substrate 13A is covered with the carrier sheet 11 until the carrier sheet 11 is peeled off from the substrate 13A. This limits the adhesion of dust or the like on the substrate 13A before the use of the adhesive body 10.

The adhesive tape 13 has a quadrangular shape as seen in the view facing the plane on which the carrier sheet 11 extends. In the example shown in Fig. 2, the adhesive tape 13 has a rectangular shape. Instead, the adhesive tape 13 may have a square shape. The carrier sheet 11 has a rectangular shape in the same manner as the adhesive tape 13. However, the shape of the carrier sheet 11 may be different from the shape of the adhesive tape 13.

The adhesive body 10 preferably satisfies at least one of the following conditions 1-1 to 1-5. The adhesive body 10 may satisfy only one of the conditions 1-1 to 1-5, may satisfy only two of the conditions 1-1 to 1-5, or may satisfy only three of the conditions 1-1 to 1-5. Alternatively, the adhesive body 10 may satisfy four conditions, or may satisfy all of the conditions 1-1 to 1-5.

(Condition 1-1) The trouser tear resistance of the carrier sheet 11 in accordance with JIS K 7128-1:1998 is less than or equal to 10 N/mm.

(Condition 1-2) The loop stiffness flexural rigidity of the carrier sheet is less than or equal to 50 mN/15 mm. In the measurement of the loop stiffness flexural rigidity, the carrier sheet 11 has a rectangular shape with a width of 15 mm and a length of 150 mm, the circumference of the loop formed by the carrier sheet 11 is 85 mm, and the compression distance is set to 20 mm.

(Condition 1-3) The substrate 13A includes a polyurethane polymer, and the tensile strength at break of the substrate 13A in accordance with JIS K 7161-1:2014 is greater than or equal to 10 MPa.

(Condition 1-4) The 180° peel strength between the carrier sheet 11 and the substrate 13A in accordance with JIS Z 0237:2009 is less than or equal to 550 mN/25 mm.

(Condition 1-5) The peel strength of the adhesive layer 13B in accordance with JIS Z 0237:2009 is greater than or equal to 1 N/25 mm.

The carrier sheet 11 is formed from a synthetic resin. The carrier sheet 11 includes, for example, a substrate sheet and a separation layer. The separation layer is laminated on the substrate sheet. When the carrier sheet 11 has the substrate sheet and the separation layer, the separation layer is in contact with the substrate 13A of the adhesive tape 13. The substrate sheet may be formed from, for example, polypropylene (PP) or polyethylene terephthalate (PET). The substrate sheet may be any one of a uniaxially stretched sheet, a biaxially stretched sheet, or an unstretched sheet. The separation layer may be formed from, for example, a silicone resin.

The carrier sheet 11 may include only the substrate sheet described above. In this case, a contact surface of the substrate sheet that is in contact with the substrate 13A may be subject to processing for facilitating peeling of the substrate 13A from the substrate sheet. The processing on the contact surface of the substrate sheet may be, for example, embossing.

The thickness of the carrier sheet 11 may be, for example, between 30 $\mu$m and 400 $\mu$m, inclusive.

The release sheet 12 is configured such that the peel strength between the release sheet 12 and the adhesive layer 13B is smaller than the peel strength between the carrier sheet 11 and the substrate 13A. The release sheet 12 is formed from a synthetic resin. The release sheet 12 includes, for example, a substrate sheet and a separation layer. The separation layer is laminated on the substrate sheet. When the release sheet 12 includes a substrate sheet and a separation layer, the separation layer is in contact with the adhesive layer 13B of the adhesive tape 13. The substrate sheet may be formed from, for example, polyethylene terephthalate (PET). The substrate sheet may be any one of a uniaxially stretched sheet, a biaxially stretched sheet, or an unstretched sheet. The separation layer may be formed from, for example, a silicone resin.

The release sheet 12 may include only the substrate sheet described above. In this case, a contact surface of the substrate sheet that is in contact with the adhesive layer 13B may be subject to processing for facilitating peeling of the adhesive layer 13B from the substrate sheet. The processing on the contact surface of the substrate sheet may be, for example, embossing.

The thickness of the release sheet 12 may be, for example, between 12 $\mu$m and 350 $\mu$m, inclusive.

The substrate 13A is formed from a synthetic resin. The synthetic resin used to form the substrate 13A may be, for example, a polyurethane resin. This provides the substrate 13A with a relatively high adhesive suitability. The thickness of the substrate 13A may be, for example, between 5 $\mu$m and 30 $\mu$m, inclusive. The substrate 13A that is formed from a polyurethane resin and is relatively thin extends well even if the external force applied to the substrate 13A for stretching the substrate 13A is relatively small. Thus, the substrate 13A has a relatively high conformity to the shape of the target to which the adhesive tape 13 will be adhered and can achieve relatively high adhesion to the adhesion target.

The substrate 13A, which has a relatively high conformity to the shape of the adhesion target, also has a relatively high conformity to the carrier sheet 11, which covers the substrate 13A. Thus, when a force is applied to the carrier sheet 11 to peel it off from the substrate 13A, the substrate 13A easily deforms following the deformation of the carrier sheet 11.

The tensile elongation at break of the substrate 13A may be, for example, greater than or equal to 130%. The tensile elongation at break can be obtained in accordance with JIS K 7161-1:2014 (ISO 527-1) "Plastics - Determination of tensile properties - Part 1: General principles" and JIS K 7127:1999 (ISO 527-3) "Plastics - Determination of tensile properties - Part 3: Test conditions for films and sheets." If the measurement object does not have a yield point, the tensile strain at break is measured. If it has a yield point, the nominal tensile strain at break is measured. The tensile elongation at break can be determined using these measurement values.

The tensile strength of the substrate 13A at 100% elongation may be, for example, less than or equal to 4 N/cm. The tensile strength at 100% elongation is a value obtained by dividing, by the width of the test piece, the magnitude of the force measured when the strain defined in JIS K 7161 - 1:2014 (ISO 527-1) "Plastics - Determination of tensile properties - Part 1: General principles" reaches a specified value (100%). The tensile strength at 100% elongation (T) (N/cm) can be determined by the following equation.

$$T = F/W$$

In this equation, F is the magnitude of the measured force (N) and W is the width of the test piece (cm).

The substrate 13A may be formed from synthetic resins other than a polyurethane resin. Examples of synthetic resins other than a polyurethane resin include a polyvinylidene fluoride resin, an ethylene-vinyl acetate copolymer resin, a polypropylene resin, and a polyethylene terephthalate resin.

In the same manner as the substrate 13A, the adhesive layer 13B is formed from a synthetic resin. The synthetic resin used to form the adhesive layer 13B may be, for example, a polyurethane resin. The thickness of the adhesive layer 13B may be, for example, between 5 μm and 25 μm, inclusive.

At least one of the substrate 13A and the adhesive layer 13B may contain an active ingredient for skin. That is, only the substrate 13A may contain an active ingredient, only the adhesive layer 13B may contain an active ingredient, or both the substrate 13A and the adhesive layer 13B may contain an active ingredient. The active ingredient may be, for example, a cosmetic ingredient, a beauty ingredient, or a pharmaceutical ingredient.

Operation

First, the user of the adhesive body 10 peels off the release sheet 12 from the adhesive layer 13B of the adhesive tape 13. Next, the user adheres the adhesive layer 13B to the target to which the adhesive tape 13 should be adhered. The adhesion target of the adhesive tape 13 is, for example, human skin, as described above. Then, the user peels off the carrier sheet 11 from the substrate 13A of the adhesive tape 13.

When the adhesive tape 13 is adhered along a relatively flat surface of the skin of a human, for example, the palm of the hand, since the carrier sheet 11 has the slit 11A, the user holds a portion of the carrier sheet 11 defining the slit 11A between the thumb and the index finger using the slit 11A of the carrier sheet 11 as a mark. Next, the user applies force to the carrier sheet 11 in the direction in which the slit 11A extends, as seen in the view facing the plane on which the carrier sheet 11 extends. Since the carrier sheet 11 has the slit 11A, the carrier sheet 11 can be easily peeled off from the substrate 13A by tearing it, compared with when the carrier sheet 11 does not have the slit 11A.

Thus, since the slit 11A of the carrier sheet 11 promotes the tearing of the carrier sheet 11, the tearing of the carrier sheet 11 easily goes beyond at least the outer edge 13AE of the substrate 13A. As a result, air is drawn into the section between the carrier sheet 11 and the 13A of the substrate through the torn part of the carrier sheet 11. Thus, even if the substrate 13A easily follows the deformation of the carrier sheet 11, the carrier sheet 11 is easily peeled off from the substrate 13A.

Further, the adhesive tape 13 may be adhered to a human finger. In this case, after the adhesive tape 13 is placed on the finger, the carrier sheet 11 is pulled together with the adhesive tape 13. As a result, the carrier sheet 11 is torn from the slit 11A. Even when the adhesive tape 13 is adhered to the finger, the carrier sheet 11 may be torn by the same method as in the case of adhering the adhesive tape 13 along a flat surface. That is, after the adhesive tape 13 is placed on the finger, a portion defining the slit 11A is held between the thumb and the index finger. Next, the user applies force to the carrier sheet 11 in the direction in which the slit 11A extends, as seen in the view facing the plane on which the carrier sheet 11 extends.

In any case, the force in the direction in which the slit 11A extends can be applied to the carrier sheet 11. Since the carrier sheet 11 has the slit 11A, the carrier sheet 11 can be easily peeled off from the substrate 13A by tearing it, compared with when the carrier sheet 11 does not have the slit 11A.

When the adhesive body 10 satisfies the condition 1-1, the carrier sheet 11 is more likely to be torn than when the trouser tear resistance exceeds 10 N/mm. Therefore, the user will feel less resistance when tearing the carrier sheet 11. In addition, the carrier sheet 11 can be split into two by tearing the carrier sheet 11, in the direction in which the slit 11A extends, from the distal end of the slit 11A to a position facing the distal end in the outer edge 11E of the carrier sheet 11.

When the adhesive body 10 satisfies the condition 1-2, the deformation of the adhesive tape 13 following the shape of the adhesion target to adhere the adhesive tape 13 to the adhesion target is less likely to be limited by the carrier sheet 11, compared with when the loop stiffness flexural rigidity is greater than 50 mN/15 mm. This facilitates the adhering of the laminate of the adhesive tape 13 and the carrier sheet 11 to the adhesion target.

In a case in which the adhesive body 10 satisfies the condition 1-3, it limits situations in which the substrate 13A of the adhesive tape 13 is torn together with the carrier sheet 11 when the carrier sheet 11 is peeled off from the adhesive tape 13, compared with a case in which the tensile strength at break of the substrate 13A is less than 10 MPa.

When the adhesive body 10 satisfies the condition 1-4, the carrier sheet 11 is easily peeled off from the substrate 13A. This limits situations in which the substrate 13A is peeled off from the adhesion target together with the carrier sheet 11.

In a case in which the adhesive body 10 satisfies the condition 1-5, it limits situations in which the adhesive tape 13 is lifted off together with the carrier sheet 11 when the carrier sheet 11 is peeled off from the adhesive tape 13, compared with a case in which the peel strength of the adhesive layer 13B is less than 1 N/25 mm. Thus, even after the carrier sheet 11 is peeled off from the adhesive tape 13, the adhesive tape 13 easily remains adhered to the adhesion target.

Test Examples

Test examples will now be described with reference to Table 1.

Test Example 1-1

A biaxially oriented polypropylene (OPP) film (FOR-MP, 40 $\mu$m thick, manufactured by Futamura Chemical Co., Ltd.) with one of two opposing surfaces being a matte surface was prepared as a carrier sheet. Then, an aqueous polyurethane (TAKELAC® WS-6021, manufactured by Mitsui Chemicals, Inc.) containing ether-based polyol was coated onto the matte surface of the OPP film. After that, a precursor layer was formed by drying the aqueous polyurethane. Then, the precursor layer was aged at room temperature to obtain a substrate having a thickness of 15 $\mu$m.

A separation film (Cerapeel® WZ, 75 $\mu$m thick, manufactured by TORAY ADVANCED FILM Co., Ltd) composed of a PET film and a separation layer that was formed from a silicone resin was prepared as a release sheet. Next, a urethane-based adhesive (SP-205, manufactured by Toyochem Co., Ltd.) as the main agent and a curing agent (T-501B, manufactured by Toyochem Co., Ltd.) were prepared. After adding the curing agent to the main agent, the main agent and the curing agent were stirred to obtain a coating solution for forming the adhesive layer. The coating solution was applied to the separation film, and then the solution was dried to obtain an adhesive layer with a thickness of 15 $\mu$m.

Then, the substrate and the adhesive layer were bonded to each other to obtain a laminate in which the OPP film, the adhesive tape, and the separation film were laminated in this order. Next, half cutting was performed on the laminate to cut out a rectangular area that extends through only the separation film and the adhesive tape. Subsequently, a portion of each of the separation film and the adhesive tape outside the section subjected to the half cutting was removed from the OPP film. Thus, the size of the separation film and the size of the adhesive tape were made smaller than the size of the OPP film. Then, a straight slit was formed in a portion of the OPP film located outside the portion where the adhesive tape was laminated. Consequently, the adhesive body of test example 1-1 was obtained.

Test Example 1-2

The adhesive body of test example 1-2 was obtained using the same method as in test example 1-1, except that the OPP film in the adhesive body of test example 1-1 was changed to a polyethylene terephthalate (PET) film (formulation 6501, 50 $\mu$m thick, manufactured by LINTEC Corporation) subjected to a release treatment.

Test Example 1-3

The adhesive body of test example 1-3 was obtained using the same method as in test example 1-1, except that the OPP film in the adhesive body of test example 1-1 was changed to a polyethylene terephthalate (PET) film (S10, 50 $\mu$m thick, manufactured by Toray Industries, Inc.).

Test Example 1-4

The adhesive body of test example 1-4 was obtained using the same method as in test example 1-1, except that the OPP film in the adhesive body of test example 1-1 was changed to a polyethylene terephthalate (PET) film (formulation 6050, 50 $\mu$m thick, manufactured by LINTEC Corporation) subjected to a release treatment.

Test Example 1-5

The adhesive body of test example 1-5 was obtained using the same method as in test example 1-1, except that the OPP film in the adhesive body of test example 1-1 was changed to a polyethylene terephthalate (PET) film (S10, 75 $\mu$m thick, manufactured by Toray Industries, Inc.).

Test Example 1-6

The adhesive body of test example 1-6 was obtained in the same manner as in test example 1-1, except that the OPP film in the adhesive body of test example 1-1 was changed to a 50 $\mu$m-thick PVDF film formed through extrusion molding using a polyvinylidene fluoride (PVDF) resin (manufactured by Arkema Inc., Kynar® FLEX2500).

Test Example 1-7

The adhesive body of test example 1-7 was obtained using the same method as in test example 1-1, except that the OPP film in the adhesive body of test example 1-1 was changed to a polypropylene (PP) film (EV130TPD, manufactured by LINTEC Corporation).

Evaluation Method

Trouser Tear Resistance

The tear resistance of the carrier sheet was measured using a method in accordance with JIS K 7128-1:1998 "Plastics - Film and sheeting - Determination of tear resistance-Part 1: Trouser tear method." In the measurement, a test piece having a width of 50 mm and a length of 150 mm was prepared from the carrier sheet of each test example. Next, a slit was formed in the test piece at one end of the test piece in the longitudinal direction, the slit starting from the center in the width direction and extending in the longitudinal direction. As a result, a first piece and a second piece separated from each other by the slit were formed in the test piece. The slit length was set to 75 mm.

Using a tensile testing machine (Autograph® AGS-X, load cell 1kN, manufactured by Shimadzu Corporation), the first and second pieces were pulled in opposite directions. Excluding the 20 mm of tear initiation and the 5 mm before tear completion, the approximate average tear resistance for the remaining 50 mm was obtained. In cases where the curve in this section is a wavy plateau, a line parallel to the horizontal axis was drawn through the center of the wavy curve, and the strength corresponding to this line was read. The tearing speed was set to 200 mm/min. The tear resistance is a value obtained by dividing the tear force (N) of the test piece by the thickness of the test piece.

Five test pieces were prepared for each test example, and the tear resistance was measured for each test piece. Then, the average value of the tear resistances of the five test pieces was calculated, and the average value was set as the tear resistance of each test example.

Loop Stiffness Flexural Rigidity

Using a loop stiffness tester (Loop Stiffness Tester® No. 581, manufactured by Toyo Seiki Seisaku-sho, Ltd.,), the loop stiffness flexural rigidity of the carrier sheet in each test example was measured.

To measure the loop stiffness flexural rigidity, first, the test piece was bent in a loop shape, and the opposite ends of the test piece in the longitudinal direction were overlapped with each other. The overlapped opposite ends were then gripped by the chuck of the loop stiffness tester. The flexural rigidity was measured by obtaining the load value detected by the load cell when the indenter of the loop stiffness tester made contact with the loop-shaped test piece and then pressed the test piece by a predetermined press amount. The measurement conditions were set as follows.

Loop (test piece) shape: width 15 mm, length 85 mm
Indenter pressing speed: 3.3 mm/sec
Indenter pressing amount: the amount that causes the distance between the indenter and the chuck to become 20 mm

Five test pieces were prepared for each test example, and the loop stiffness flexural rigidity was measured for each test piece. Then, the average value of the loop stiffness flexural rigidities of the five test pieces was calculated, and the average value was set as the loop stiffness flexural rigidity of each test example.

Tensile Strength at Break

To measure the tensile strength at break of the substrate of each test example, first, the substrate was cut into a dumbbell shape (test piece type 5) using a method in accordance with JIS K 7127:1999 "Plastics - Determination of tensile properties - Part 3: Test conditions for films and sheets," thereby creating a test piece. Next, the tensile strength at break of each test piece was measured using a method in accordance with JIS K 7161-1:2014 "Plastics - Determination of tensile properties - Part 1: General principles." The tensile strength at break was measured using a universal tester (Autograph AGS-X load cell 5kN, manufactured by Shimadzu Corporation). The tensile speed was set to 300 mm/min, and the gauge length was set to 25mm.

Five test pieces were prepared for each test example, and the tensile strength at break was measured for each test piece. Then, the average value of the tensile strengths at break of the five test pieces was calculated, and the average value was set as the tensile strength at break of each test example.

180° Peel Strength

The peel strength of the substrate from the carrier sheet was measured using a method in accordance with JIS Z 0237:2009 "Testing methods of pressure-sensitive adhesive tapes and sheets." The peel strength was measured using a universal tester (Autograph AGS-X load cell 5kN, manufactured by Shimadzu Corporation). To measure the peel strength, the adhesive body of each test example was prepared, and a test piece having a width of 25 mm was cut out from the adhesive body. Then, the substrate was fixed to the universal tester, and the strength when peeling off the carrier sheet from the substrate at 180° was calculated.

Five test pieces were prepared for each test example, and the 180° peel strength was measured for each test piece. Then, the average value of the 180° peel strengths of the five test pieces was calculated, and the average value was set as the 180° peel strength of each test example.

Peel Strength

The peel strength of the adhesive layer was measured using a method in accordance with JIS Z 0237:2009 "Testing methods of pressure-sensitive adhesive tapes and sheets." The peel strength was measured using a universal tester (Autograph AGS-X load cell 5kN, manufactured by Shimadzu Corporation). To measure the peel strength, a SUS plate with a BA surface finish, formed from SUS304, was prepared. Further, a test piece having a width of 25 mm was prepared from the adhesive layer of each test example. After bringing the adhesive layer into contact with the SUS plate, the adhesive layer was pressed onto the SUS plate using a 2-kg rubber roller. Next, the strength when peeling off the test piece from the SUS plate by 180° was calculated using the universal tester. The test speed of the universal tester was set to 300 mm/min.

Five test pieces were prepared for each test example, and the peel strength was measured for each test piece. Then, the average value of the peel strengths of the five test pieces was calculated, and the average value was set as the peel strength of each test example.

Adhering to Skin and Peeling of Carrier Sheet

In each test example, the size of the adhesive tape was set to 10 cm square, and the size of the carrier sheet was set to 12 cm square. Then, only one straight slit having a length of 1 cm from the outer edge of the carrier sheet was formed in the carrier sheet. Then, the adhesion to the skin of the arm of the human body when the adhesive body of each test example was adhered, and the peeling of the carrier sheet after the adhesive tape was adhered to the skin, were evaluated. For each test example, five adhesive bodies were evaluated.

Evaluation Results

The evaluation results will now be described with reference to Table 1.

Table 1

|  | Tear Resistance (N/mm) | Flexural Rigidity (mN/15mm) | Tensile Strength at Break (MPa) | 180° Peel Strength (mN/25mm) | Peel Strength (N/25mm) | Adhesion to Skin | Peel of Carrier Sheet |
|---|---|---|---|---|---|---|---|
| Test Example 1-1 | 4.3 | 1.0 | 32.8 | 85.1 | 3.5 | ○ | ○ |
| Test Example 1-2 | 7.6 | 43. 1 | 25.6 | 81.0 | 3.6 | ○ | ○ |
| Test Example 1-3 | 7.8 | 36.8 | 28.3 | 492.0 | 4.1 | ○ | ○ |
| Test Example 1-4 | 7.6 | 42.5 | 26.1 | 579.0 | 3.6 | ○ | Δ |
| Test Example 1-5 | 9.7 | 115.4 | 31.5 | 507.6 | 3.8 | Δ | ○ |
| Test Example 1-6 | 28.3 | 11.0 | 31.1 | 716.9 | 3.5 | ○ | Δ |
| Test Example 1-7 | 41.9 | 731.0 | 32.6 | 121.0 | 3.4 | Δ | Δ |

As shown in Table 1, it was observed that the tear resistance of the carrier sheet was 4.3 N/mm in test example 1-1, 7.6 N/mm in test example 1-2, 7.8 N/mm in test example 1-3, and 7.6 N/mm in test example 1-4. Additionally, it was observed that the tear resistance of the carrier sheet was 9.7 N/mm in test example 1-5, 28.3 N/mm in test example 1-6, and 41.9 N/mm in test example 1-7.

It was observed that the flexural rigidity of the carrier sheet was 7.0 mN/15 mm in test example 1-1, 43.1 mN/15 mm in test example 1-2, 36.8 mN/15 mm in test example 1-3, and 42.5 mN/mm in test example 1-4. It was observed that the flexural rigidity of the carrier sheet was 115.4 mN/mm in test example 1-5, 11.0 mN/mm in test example 1-6, and 731.0 mN/15 mm in test example 1-7.

It was observed that the tensile strength at break of the substrate was 32.8 MPa in test example 1-1, 25.6 MPa in test example 1-2, 28.3 MPa in test example 1-3, and 26.1 MPa in test example 1-4. It was observed that the tensile strength at break of the substrate 13A was 31.5 MPa in test example 1-5, 31.1 MPa in test example 1-6, and 32.6 MPa in test example 1-7.

It was observed that the 180° peel strength between the carrier sheet and the substrate was 85.1 mN/25 mm in test example 1-1, 81.0 mN/25 mm in test example 1-2, and 492.0 mN/25 mm in test example 1-3. It was observed that the 180° peel strength between the carrier sheet and the substrate was 579.0 mN/25 mm in test example 1-4, and 507.6 mN/25 mm in test example 1-5. It was observed that the 180° peel strength between the carrier sheet and the substrate was 716.9 mN/25 mm in test example 1-6, and 121.0 mN/25 mm in test example 1-7.

It was observed that the peel strength of the adhesive layer was 3.5 N/25 mm in test example 1-1, 3.6 N/25 mm in test example 1-2, 4.1 N/25 mm in test example 1-3, and 3.6 N/25 mm in test example 1-4. It was observed that the peel strength of the adhesive layer was 3.8 N/25 mm in test example 1-5, 3.5 N/25 mm in test example 1-6, and 3.4 N/25 mm in test example 1-7.

When the adhesive tape of each test example were adhered to the skin of the arm, it was observed that in all five adhesive tapes from test examples 1-1 to 1-4, and 1-6, it was possible to adhere the adhesive tape without causing wrinkles in the adhesive tape. That is, it was observed that the adhesion evaluation result was "o" (satisfactory) for the adhesive bodies of test examples 1-1 to 1-4 and 1-6.

For at least one of the adhesive bodies of test examples 1-5 and 1-7, it was observed that the adhesive tape was wrinkled when adhered. That is, it was observed that the adhesion evaluation result was "△" (poor) for the adhesive bodies of test examples 1-5 and 1-7. This result indicates that when the loop stiffness flexural rigidity of the carrier sheet is less than or equal to 43.1 mN/15 mm, the carrier sheet's conformity to the shape of the skin is enhanced, thereby suppressing the formation of wrinkles in the adhesive tape.

Additionally, in the adhesive bodies of test examples 1-1 through 1-3 and 1-5, it was observed that when peeling the carrier sheet from the substrate of the adhesive tape, the carrier sheet could be torn along the slit, thereby allowing the carrier sheet to be divided into two parts. That is, it was observed that the peeling evaluation result was "o" for the adhesive bodies of test examples 1-1 to 1-3 and 1-5.

In the adhesive bodies of test example 1-4, it was observed that when peeling the carrier sheet from the substrate of the adhesive tape, the tearing of the carrier sheet could proceed. In at least one of these adhesive bodies, it was observed that due to the strong adhesion between the carrier sheet and the substrate, it was difficult for air to be drawn into the section between the carrier sheet and the substrate at the torn position of the carrier sheet. Further, when peeling the carrier sheet from the substrate, it was observed that the adhesive tape lifted along with the carrier sheet, causing at least part of the adhesive tape to peel off from the skin. That is, it was observed that the peeling evaluation result was "△" for the adhesive body of test example 1-4.

The comparison of the adhesive bodies of test examples 1-1 through 1-3 and 1-5 with the adhesive body of test example 1-4 indicates that when the 180° peel strength between the carrier sheet and the substrate is less than or equal to 507.6 mN/25 mm, the carrier sheet is likely to peel off from the substrate.

Further, in the adhesive body of test example 1-7, it was observed that when peeling the carrier sheet from the substrate of the adhesive tape, the entire carrier sheet could not be torn or the resistance when tearing the carrier sheet was significant. That is, it was observed that the evaluation result was "△" for the adhesive body of test example 1-7. However, in the adhesive body of test example 1-7, it was observed that since the carrier sheet had a slit, the carrier sheet could be torn to a position inside the outer edge of the substrate. Moreover, it was observed that by drawing air into the section between the carrier sheet and the substrate from the position where the carrier sheet was torn, the carrier sheet could be easily peeled off from the substrate.

The comparison of the adhesive bodies of test examples 1-1 through 1-3 and 1-5 with the adhesive body of test example 1-7 indicates that when the tear resistance of the carrier sheet is less than or equal to 9.7 N/mm, the carrier sheet is torn easily.

Further, in the adhesive body of test example 1-6, it was observed that when peeling the carrier sheet from the substrate of the adhesive tape, the entire carrier sheet could not be torn or the resistance when tearing the carrier sheet was significant. That is, it was observed that the evaluation result was "△" for the adhesive body of test example 1-6.

In the adhesive body of test example 1-6, it was observed that since the carrier sheet had a slit, the carrier sheet could be torn to a position inside the outer edge of the substrate. In the same manner as the adhesive body of test example 1-4, in the adhesive body of test example 1-6, in at least one adhesive body, it was observed that due to the strong adhesion between the carrier sheet and the substrate, it was difficult for air to be drawn into the section between the carrier sheet and the substrate at the torn position of the carrier sheet. Further, when peeling the carrier sheet from the substrate, it was observed that the adhesive tape lifted along with the carrier sheet, causing at least part of the adhesive tape to peel off from the skin.

As described above, the adhesive body of the first embodiment provides the following advantages.

(1□1) Since the carrier sheet 11 has the slit 11A, it is easier to tear the carrier sheet 11 and thereby peel the carrier sheet 11 from the substrate 13A compared with when the carrier sheet 11 does not have the slit 11A.

(1-2) When the trouser tear resistance of the carrier sheet 11 is less than or equal to 10 N/mm, the carrier sheet is torn more easily.

(1-3) When the loop stiffness flexural rigidity of the carrier sheet 11 is less than or equal to 50 mN/15 mm, the deformation of the adhesive tape 13 following the shape of the adhesion target is less likely to be limited by the carrier sheet 11.

(104) In a case in which the tensile strength at break of the substrate 13A is greater than or equal to 10 MPa, it limits situations in which the substrate 13A of the adhesive tape 13 is torn together with the carrier sheet 11 when the carrier sheet 11 is peeled off from the adhesive tape 13.

(1-5) In a case I which the 180° peel strength between the carrier sheet 11 and the substrate 13A is less than or equal to 550 mN/25 mm, the carrier sheet 11 is easily peeled off from the substrate 13A when the carrier sheet 11 is peeled off from the substrate 13A.

(6) In a case in which the peel strength of the adhesive layer 13B is greater than or equal to 1 N/25 mm, it limits situations in which the adhesive tape 13 is lifted off together with the carrier sheet 11 when the carrier sheet 11 is peeled off from the adhesive tape 13.

Modifications of First Embodiment

The above-described first embodiment may be modified as follows.

Slit

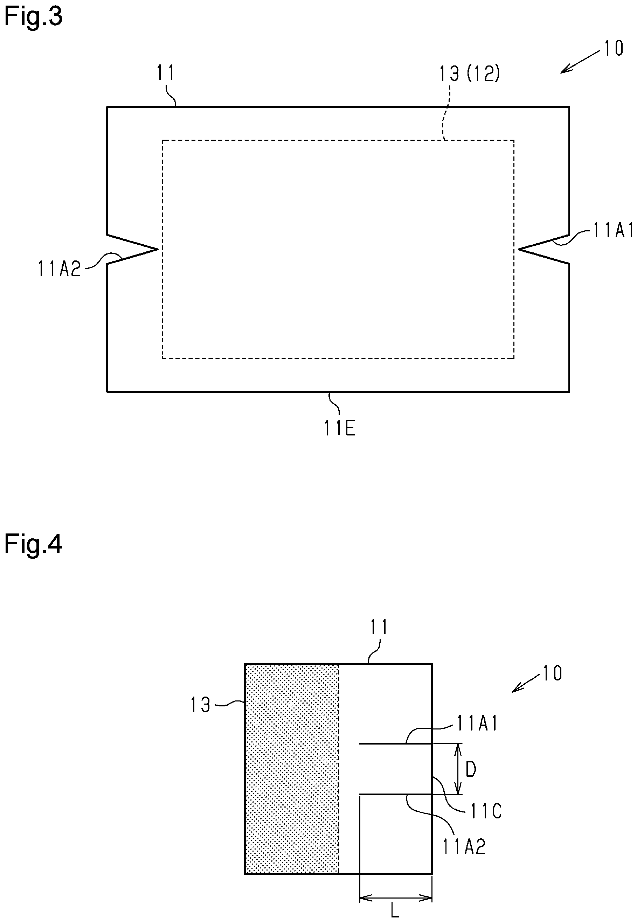

As shown in Fig. 3, the adhesive body 10 may have two or more slits. In the example shown in Fig. 3, the adhesive body 10 includes a first slit 11A1 and a second slit 11A2. The first slit 11A1 and the second slit 11A2 are arranged on the outer edge 11E of the carrier sheet 11 so as to face each other. The first slit 11A1 and the second slit 11A2 are opposed to each other with the adhesive tape 13 located in between, as seen in the view facing the plane on which the carrier sheet 11 extends. The distal end of the first slit 11A1 and the distal end of the second slit 11A2 face each other with the adhesive tape 13 located in between.

This adhesive body 10 provides the following advantage.

(1-7) For example, when the carrier sheet 11 is torn from the first slit 11A1, the tearing of the carrier sheet 11 reaches the second slit 11A2, thereby splitting the carrier sheet 11 into two parts. Therefore, compared with when the second slit 11A2 is not located at a position facing the first slit 11A1, the carrier sheet 11 is easier to tear.

The first slit 11A1 and the second slit 11A2 do not have to be arranged so as to face each other. Even in this case, by having two or more slits in the carrier sheet 11, it is possible to choose from two or more positions to start tearing the carrier sheet 11, compared with when the carrier sheet 11 has only one slit.

The slit is not limited to an I-notch. For example, like the slits 11A1 and 11A2 shown in Fig. 3, the slits may be V-notches. Alternatively, the slits may be U-notches or UV-notches. When the carrier sheet 11 has two or more slits, the slits may include a slit with a first shape and a slit with a second shape that is different from the first shape.

The first slit 11A1 and the second slit 11A2 may be arranged as shown in Figs. 4 to 8. In Figs. 4 to 8, dots are added to the adhesive tape 13 to clearly differentiate the shape of the carrier sheet 11 from that of the adhesive tape 13.

The adhesive body 10 shown in Fig. 4 includes the first slit 11A1 and the second slit 11A2. The first slit 11A1 and the second slit 11A2 are spaced apart from each other in an arrangement direction, and each slit 11A1, 11A2 extends in an extending direction that intersects the arrangement direction. The arrangement direction is an example of a first direction, and the extending direction is an example of a second direction. Each slit 11A1, 11A2 has a straight shape extending in the extending direction. Length L of each slit 11A1, 11A2 in the extending direction may be, for example, greater than or equal to 5 mm. To make it easier to grip a portion of the carrier sheet 11 located between the two slits 11A1 and 11A2, interval D between the slits 11A1 and 11A2 in the arrangement direction may be less than, for example, 50% of the length of the carrier sheet 11 in the arrangement direction. To make it easier to grip a portion of the carrier sheet 11 located between the two slits 11A1 and 11A2, interval D between the slits 11A1 and 11A2 in the arrangement direction may be less than, for example, between 2 mm and 1 cm, inclusive.

The first slit 11A1 and the second slit 11A2 are located on the same side with respect to the end of the adhesive tape 13 in the carrier sheet 11. The adhesive tape 13 is not located between the first slit 11A1 and the second slit 11A2. The first slit 11A1 and the second slit 11A2 define a grip portion 11C in the carrier sheet 11. The grip portion 11C is located between the first slit 11A1 and the second slit 11A2 and extends in the extending direction.

In the example shown in Fig. 4, the carrier sheet 11 and the adhesive tape 13 each have a rectangular shape. In the arrangement direction, the adhesive tape 13 has the same length as the carrier sheet 11. In the extending direction, the length of the adhesive tape 13 is shorter than the length of the carrier sheet 11. In the example shown in Fig. 4, the basal end of the first slit 11A1 and the basal end of the second slit 11A2 are located on the same side.

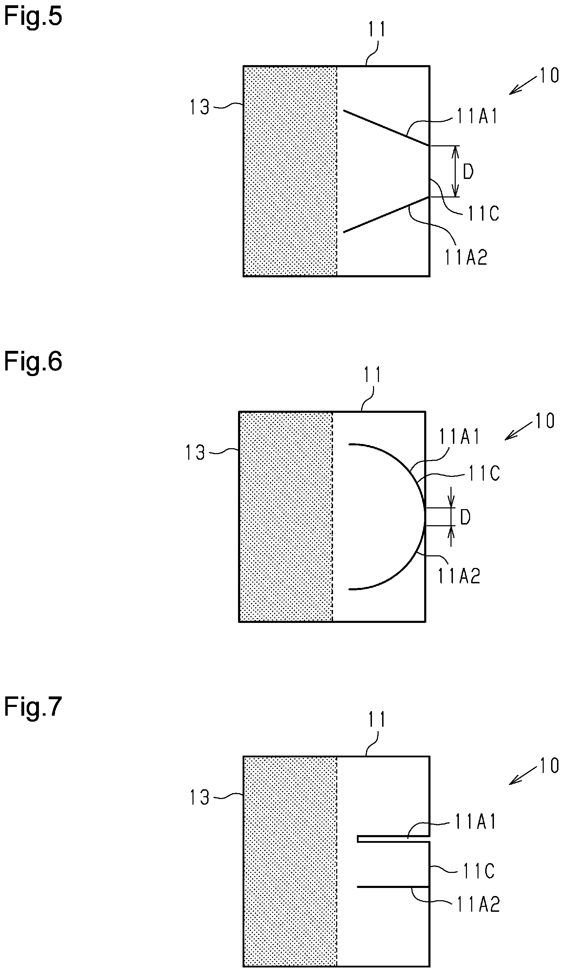

As shown in Fig. 5, the first slit 11A1 and the second slit 11A2 may be configured such that interval D between the slits 11A1 and 11A2 increases from the basal end to the distal end of each slit 11A1, 11A2. Each slit 11A1, 11A2 is configured to diverge from each other as the distance from the basal end increases. Each slit 11A1, 11A2 has a straight shape. In the example shown in Fig. 5, the carrier sheet 11 has a rectangular shape. Thus, each slit 11A1, 11A2 extends in a direction intersecting, at an angle smaller than a right angle, the side on which the basal end of each slit 11A1, 11A2 is located.

In the same manner as the adhesive body 10 shown in Fig. 5, in the adhesive body 10 shown in Fig. 6, the first slit 11A1 and the second slit 11A2 may be configured such that interval D between the slits 11A1 and 11A2 increases from the basal end to the distal end of each slit 11A1, 11A2. Each slit 11A1, 11A2 is configured to diverge from each other as the distance from the basal end increases. Each slit 11A1, 11A2 has an arcuate shape. In the example shown in Fig. 6, the carrier sheet 11 has a rectangular shape. Thus, each slit 11A1, 11A2 extends in a direction intersecting, at an angle smaller than a right angle, the side on which the basal end of each slit 11A1, 11A2 is located.

As shown in Fig. 7, the shape of the first slit 11A1 may be different from the shape of the second slit 11A2. In the example shown in Fig. 7, the first slit 11A1 is wider than the second slit 11A2 in the arrangement direction. The shape of the first slit 11A1 only needs to be different from the shape of the second slit 11A2. Thus, the first slit 11A1 may be one selected from the group consisting of an I notch, a V notch, a U notch, and a UV notch, and the second slit 11A2 may be selected from the same group and have a shape different from that of the first slit 11A1.

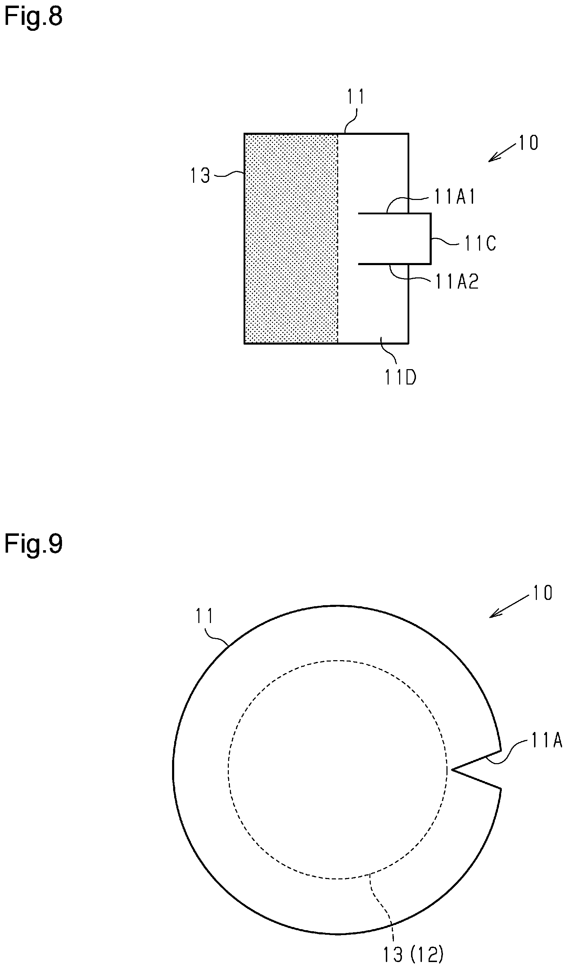

As shown in Fig. 8, the carrier sheet 11 includes a main body 11D and a grip portion 11C, and the first slit 11A1 and the second slit 11A2 separate the grip portion 11C from the main body 11D. The grip portion 11C is longer than each slit 11A1, 11A2 in the extending direction. A portion of the grip portion 11C that is not located between the first slit 11A1 and the second slit 11A2 protrudes from the outer edge of the main body 11D in the extending direction.

Thus, when the adhesive body 10 includes the grip portion 11C located between the first slit 11A1 and the second slit 11A2, the following advantage is provided.

(1-8) The grip portion 11C located between the first slit 11A1 and the second slit 11A2 can be gripped to apply force for tearing the carrier sheet 11 to the carrier sheet 11. Thus, the carrier sheet 11 can be torn more easily.

Adhesive Tape

As shown in Fig. 9, the adhesive tape 13 may have a circular shape as seen in the view facing the plane on which the carrier sheet 11 extends. In the example shown in Fig. 9, the carrier sheet 11 is circular in the same manner as the adhesive tape 13. The carrier sheet 11 and the adhesive tape 13 have circular shapes concentric with each other. Even when the adhesive tape 13 is circular, the carrier sheet 11 may be quadrangular.

In the example shown in Fig. 9, the slit 11A of the carrier sheet 11 is a V-notch. Instead, the slit 11A may be any one of an I-notch, a U-notch, and a UV-notch.

As shown in Figs. 1 to 3, when the adhesive tape 13 has a rectangular shape, the carrier sheet 11 may have a circular shape. Even in this case, since the carrier sheet 11 has a slit, the advantages equivalent to the above-described (1) are provided.

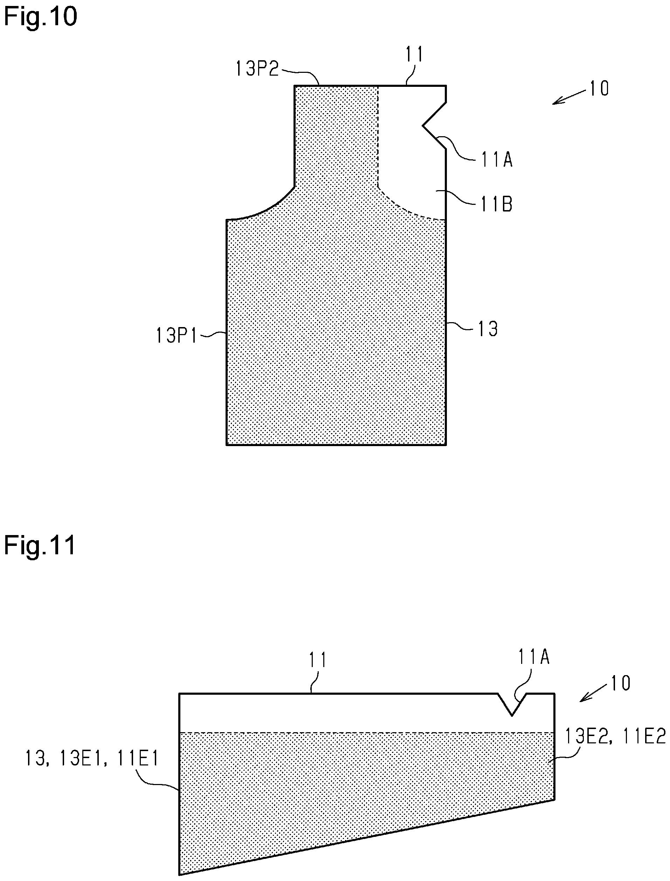

As shown in Fig. 10, the adhesive tape 13 may have a shape that will be described below. In Fig. 10, dots are added to the adhesive tape 13 to clearly differentiate the shape of the carrier sheet 11 from that of the adhesive tape 13.

As shown in Fig. 10, the adhesive tape 13 includes a first portion 13P1 and a second portion 13P2 as seen in the view facing the plane on which the carrier sheet 11 extends. The first portion 13P1 and the second portion 13P2 are arranged in an extending direction. The first portion 13P1 has a substantially quadrangular shape, and has a shape tapered toward the second portion 13P2 at an end connected to the second portion 13P2. In other words, the first portion 13P1 has a shape where one of the four corners of the rectangle is notched by the side to which the second portion 13P2 is connected.

The second portion 13P2 has a rectangular shape. The second portion 13P2 has a smaller area than the first portion 13P1. The second portion 13P2 is connected to a portion including the center of the first portion 13P1 in the width direction, which is orthogonal to the extending direction.

The substrate 13A and the adhesive layer 13B, included in the adhesive tape 13, have the same shape as that of the adhesive tape 13 in a plan view. The release sheet 12 has the same shape as that of the adhesive tape 13.

The carrier sheet 11 is sized and shaped so as to cover the entirety of the first portion 13P1 and the second portion 13P2 of the adhesive tape 13. The carrier sheet 11 has a substantially rectangular shape. Further, the carrier sheet 11 has a shape in which one of the two corners arranged in the width direction in the rectangular shape is notched along the outer edge of the adhesive tape 13. The carrier sheet 11 includes a non-overlapping portion 11B, which does not overlap the adhesive tape 13. As seen in the view facing the plane on which the carrier sheet 11 extends, the non-overlapping portion 11B is adjacent to the second portion 13P2 of the adhesive tape 13 in the width direction. The non-overlapping portion 11B has the slit 11A extending from the edge of the carrier sheet 11 toward the adhesive tape 13.

The method for using the adhesive body 10 will now be described. The adhesive body 10 has a shape preferable for adhering to a protrusion. Examples of the protrusion include a human fingertip. The using method in a case where the adhesion target is a human fingertip will now be described.

To use the adhesive body 10, first, the release sheet 12 is released from the adhesive layer 13B. Next, a finger is placed on the adhesive layer 13B of the adhesive tape 13 such that the finger extends in the extending direction and the fingertip is located at the end of the first portion 13P1 connected to the second portion 13P2. Then, the carrier sheet 11 is torn in the width direction from the slit 11A, and the second portion 13P2 of the adhesive tape 13 is stretched at the part where the carrier sheet 11 is torn. Then, the fingertip is covered with the stretched second portion 13P2.

Then, portions of the first portion 13P1 of the adhesive tape 13 that are on opposite sides of the finger in the width direction are wound around the finger. This allows the adhesive tape 13 to be adhered to the finger so as to cover the finger.

As shown in Fig. 11, the adhesive tape 13 may have a shape that will be described below.

As shown in Fig. 11, the adhesive tape 13 has a trapezoidal shape as seen in the view facing the plane on which the carrier sheet 11 extends. In the adhesive tape 13, the length in the extending direction is longer than the length in the width direction. The extending direction is orthogonal to the width direction. The length of the adhesive tape 13 in the width direction monotonically decreases from a first end 13E1 toward a second end 13E2 in the extending direction.

In the adhesive tape 13, the two sides extending in the extending direction include a first side. The first side is the boundary between the adhesive tape 13 and the portion of the carrier sheet 11 where the adhesive tape 13 is not located. The first side does not change its position in the width direction. The two sides extending in the extending direction include a second side, which is different from the first side. The second side changes its position in the width direction from the first end to the second end in the extending direction.

In the same manner as the adhesive tape 13, the carrier sheet 11 has a quadrangular shape as seen in the view facing the plane on which the carrier sheet 11 extends. In the carrier sheet 11, the length in the extending direction is longer than the length in the width direction. The length of the carrier sheet 11 in the width direction monotonically decreases from a first end 11E1 toward a second end 11E2 in the extending direction. The first end 11E1 of the carrier sheet 11 in the extending direction is overlapped with the first end 13E1 of the adhesive tape 13 in the extending direction. The second end 11E2 of the carrier sheet 11 in the extending direction is overlapped with the second end 13E2 of the adhesive tape 13 in the extending direction.

In the carrier sheet 11, the two sides extending in the extending direction include a first side. The first side is an edge of the carrier sheet 11 at a portion of the carrier sheet 11 where the adhesive tape 13 is not located. The first side does not change its position in the width direction. The first side of the carrier sheet 11 is parallel to the first side of the adhesive tape 13. The two sides extending in the extending direction include a second side, which is different from the first side. The second side changes its position in the width direction from the first end to the second end in the extending direction. The second side of the carrier sheet 11 is overlapped with the second side of the adhesive tape 13.

The slit 11A is positioned closer to the second end 11E2 than to the center in the extending direction of the carrier sheet 11. In other words, in the extending direction, the distance from the slit 11A to the second end 11E2 of the carrier sheet 11 is shorter than the distance from the center of the carrier sheet 11 to the second end 11E2 of the carrier sheet 11. In the example shown in Fig. 11, the slit 11A is located relatively close to the second end 11E2 of the carrier sheet 11 in the extending direction.

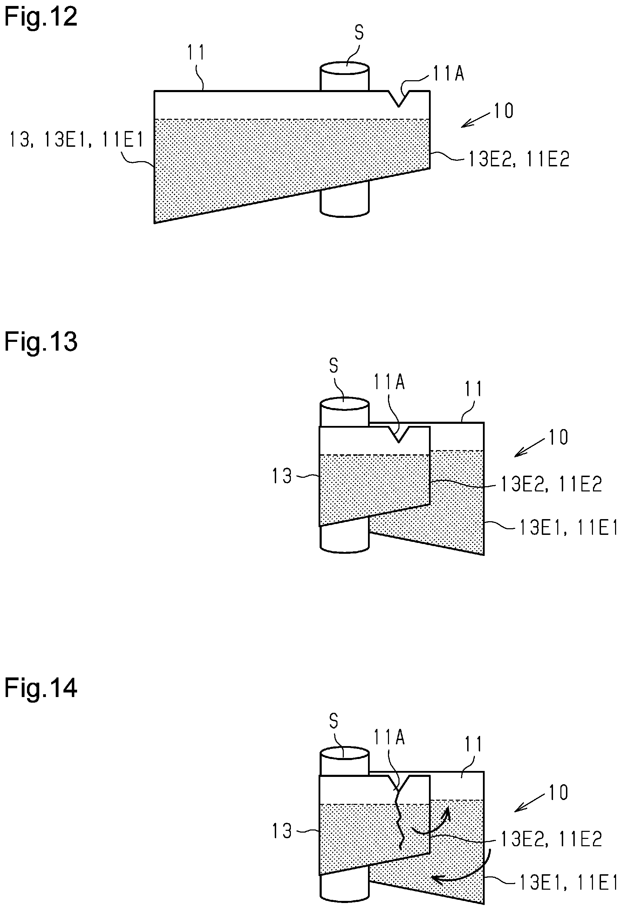

The steps that adhere the adhesive tape 13, included in the adhesive body 10, to an adhesion target will now be described with reference to Figs. 12 to 14. The adhesive tape 13 has a shape suitable for being applied to an adhesion target having a rod shape extending in one direction.

To adhere the adhesive tape 10 to an adhesion target S, first, the user of the adhesive body 10 peels off the release sheet 12 from the adhesive layer 13B of the adhesive tape 13.

Next, as shown in Fig. 12, the direction in which the adhesion target S extends is aligned with the width direction of the adhesive tape 13 with the adhesive layer 13B facing the adhesion target S. Then, the adhesion target S is moved to a substantially central position in the longitudinal direction of the adhesive tape 13.

Subsequently, as shown in Fig. 13, part of the adhesive layer 13B is temporarily fixed to the adhesion target S. In this state, the adhesive body 10 is folded such that the first end 13E1 and the second end 13E2 of the adhesive tape 13 in the longitudinal direction face each other.

Then, as shown in Fig. 14, the second end 13E2 of the adhesive tape 13 is gripped, thereby producing force to tear the carrier sheet 11 from the slit 11A to the carrier sheet 11. This causes the carrier sheet 11 to be torn in the width direction of the carrier sheet 11. Since the slit 11A is located closer to the second end 11E2, the carrier sheet 11 is torn more easily compared with when the slit 11A is located closer to the first end 11E1. Subsequently, the first end 13E1 of the adhesive tape 13 is pulled in a direction away from the adhesion target S to stretch the adhesive tape 13. Further, the section of the adhesive tape 13 from the second end 13E2 to the portion temporarily fixed to the adhesion target S is adhered to the adhesion target S while peeling off the carrier sheet 11 from the substrate 13A. Thereafter, the section of the adhesive tape 13 from the first end 13E1 to the adhesion target S is adhered to the adhesion target S so as to cover the portion of the adhesive tape 13 that has already been adhered to the adhesion target S. Since the first end 13E1 is wider than the second end 13E2, even if the adhesive tape 13 decreases monotonously, a decrease in the size of the area covering the adhesion target S is limited.

This adhesive body 10 provides the following advantage.

(1-9) It is possible for the carrier sheet 11 to be easily torn while ensuring an area for covering the adhesion target S with the adhesive tape 13.

In the above-described adhesive body 10, the adhesive tape 13 and the carrier sheet 11 may each have a rectangular shape.

Number of Adhesive Tapes

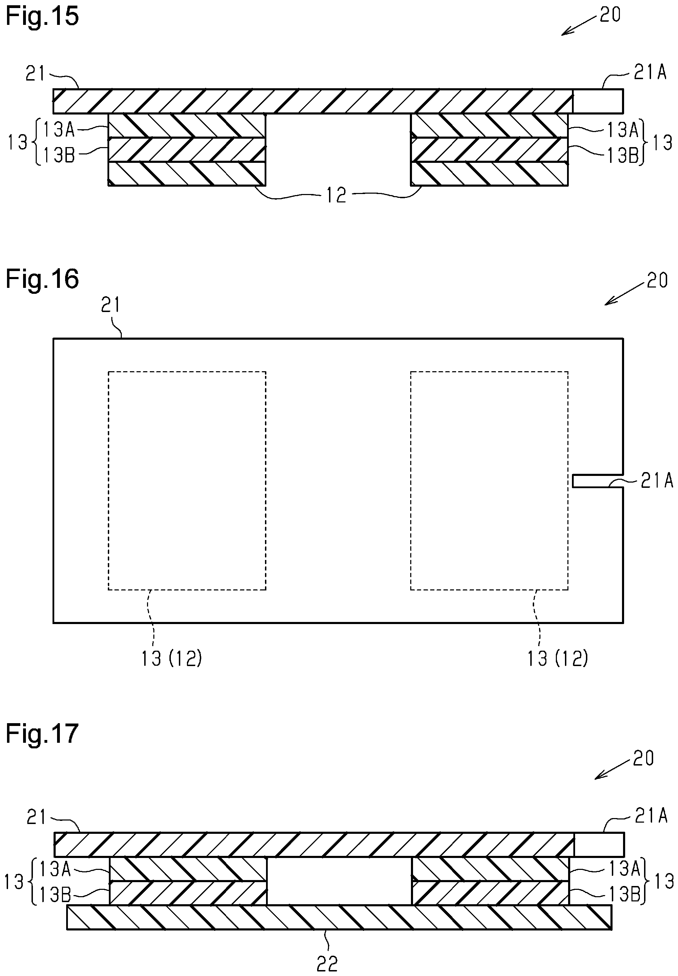

As shown in Figs. 15 to 19, the adhesive body may include two or more adhesive tapes.

For example, as shown in Fig. 15, an adhesive body 20 includes one carrier sheet 21, two adhesive tapes 13, and two release sheets 12. That is, in the adhesive body 20, two adhesive tapes 13 are attached to one carrier sheet 21. The adhesive layer 13B of each adhesive tape 13 is covered with one release sheet 12, and the release sheet 12 covering one adhesive layer 13B is different from the release sheet 12 covering the other adhesive layer 13B.

As shown in Fig. 16, the two adhesive tapes 13 are separated from each other as seen in the view facing the plane on which the carrier sheet 21 extends. The carrier sheet 21 has one slit 21A. In the example shown in Fig. 16, the gap between one of the adhesive tapes 13 and the slit 21A is smaller than the gap between the other adhesive tape 13 and the slit 21A. The distal end of the slit 21A is located relatively close to one adhesive tape 13 as seen in the view facing the plane on which the carrier sheet 21 extends. In the example shown in Fig. 16, the adhesive body 20 may have two or more slits 21A. In the example shown in Fig. 16, the slit 21A is an I-notch. Instead, the slit 21A may be any one of a V-notch, a U-notch, and a UV-notch.

As shown in Fig. 17, the adhesive body 20 may include one carrier sheet 21, two adhesive tapes 13, and one release sheet 22. That is, the adhesive body 20 may include one release sheet 22 that is shared by the two adhesive tapes 13. Thus, the two adhesive layers 13B can be exposed at the same time by peeling off one release sheet 22. The carrier sheet 21 may have two or more slits 21A.

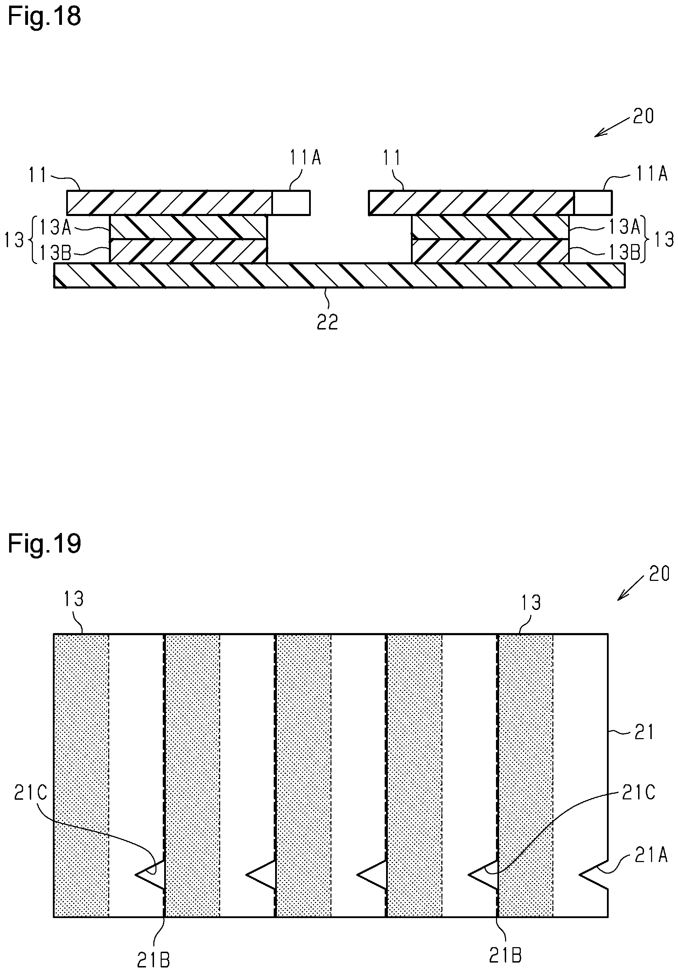

As shown in Fig. 18, the adhesive body 20 may include two carrier sheets 11, two adhesive tapes 13, and one release sheet 22. That is, while the adhesive body 20 includes one release sheet 22 that is shared by the two adhesive tapes 13, the substrate 13A of each adhesive tape 13 is covered with one carrier sheet 11 and the carrier sheet 11 covering one substrate 13A is different from the carrier sheet 11 covering the other substrate 13A. Each of the carrier sheets 11 has one slit 11A. Each carrier sheet 11 may include more than one slit 11A.

Fig. 19 is a plan view of the adhesive body 20 in which multiple adhesive tapes 13 are attached to one carrier sheet 21, as seen in the view facing the plane on which the carrier sheet 21 extends. In Fig. 19, dots are added to the adhesive tapes 13 to clearly differentiate the carrier sheet 11 from the adhesive tapes 13.

As shown in Fig. 19, the adhesive body 20 includes multiple adhesive tapes 13 attached to one carrier sheet 21. In the same manner as the adhesive body 20 described with reference to Fig. 15, the adhesive layer 13B of each adhesive tape 13 is covered with one release sheet 12. The carrier sheet 21 includes the slit 21A. The slit 21A extends toward the adhesive tape 13 from a portion of the edge of the carrier sheet 21 that does not overlap each adhesive tape 13, and does not reach the outer edge of the adhesive tape 13.

As seen in the view facing the plane on which the carrier sheet 21 extends, the portion of the carrier sheet 21 that does not overlap each adhesive tape 13 is aligned with the adhesive tape 13 in a first direction. Each adhesive tape 13 has a shape extending in a second direction that is orthogonal to the first direction.

The carrier sheet 21 includes perforations 21B. Each perforation 21B is configured such that part of the carrier sheet 21 and one adhesive tape 13 can be cut off from the adhesive body 20. In other words, each perforation 21B is configured such that a unit carrier, which is a group of portions of the carrier sheet 21 that do not overlap the adhesive tape 13 and portions of the carrier sheet 21 that overlap the adhesive tape 13, can be cut off from other portions of the carrier sheet 21. Therefore, the perforation 21B is located at the boundary between one of the unit carriers and another of the unit carriers adjacent thereto. Each perforation 21B includes through-holes arranged in the second direction. Each through-hole extends through the carrier sheet 21 in the thickness direction of the carrier sheet 21.

The carrier sheet 21 includes through portions 21C. Each through portion 21C extends through the carrier sheet 21 in the thickness direction of the carrier sheet 21. Each through portion 21C is located at the boundary between one of the unit carriers and another of the unit carriers adjacent thereto so as to divide one perforation 21B in the second direction. Each through portion 21C has a shape tapered from the perforation 21B toward a portion of the carrier sheet 21 that does not overlap the adhesive tape 13.

When the perforation 21B divided by the through portion 21C breaks, each through portion 21C is located at the edge of the broken carrier sheet 21. That is, a notch is formed from the through portion 21C by the breakage of the perforation 21B. This allows the user of the adhesive body 20 to tear the unit carrier using the notch, formed from the through portion 21C, when detaching the unit carrier from one adhesive tape 13.

Second Embodiment

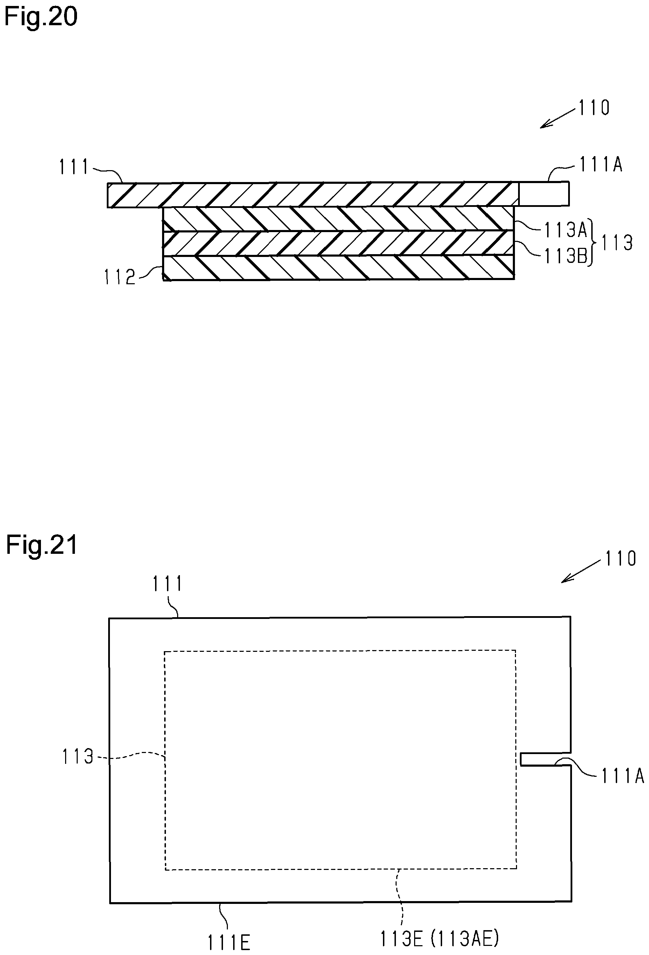

The adhesive body according to a second embodiment will now be described with reference to Figs. 20 to 26.

Structure

The structure of the adhesive body will now be described with reference to Figs. 20 and 21.

Fig. 20 shows the structure of the adhesive body in a cross-section that is orthogonal to the plane in which the adhesive body extends.

As shown in Fig. 20, an adhesive body 110 includes a carrier sheet 111, a release sheet 112, and an adhesive tape 113. The adhesive tape 113 is located between the carrier sheet 111 and the release sheet 112. The adhesive tape 113 includes a substrate 113A and an adhesive layer 113B. The substrate 113A is in contact with the carrier sheet 111. The substrate 113A has a higher tensile elongation at break than the carrier sheet 111 and the release sheet 112. The adhesive layer 113B is in contact with the release sheet 112. The adhesive layer 113B is adhered to a living body surface after the release sheet 112 is peeled off from the adhesive layer 113B. In the present embodiment, the adhesive layer 113B is laminated on the substrate 113A.

The living body surface to which the adhesive layer 113B is adhered is, for example, human skin, but may be body surfaces of animals other than humans, for example, mammals other than humans. In the present embodiment, an exemplary case in which the adhesive layer 113B is adhered to human skin will be described.

Fig. 21 shows a structure of the adhesive body 110 as seen in a view facing a plane on which the carrier sheet 111 extends.

As shown in Fig. 21, the carrier sheet 111 has a larger area than the adhesive tape 113, and the adhesive tape 113 is located within the carrier sheet 111 as seen in a view facing a plane on which the carrier sheet 111 extends. In the present embodiment, the area of the carrier sheet 111 is larger than the area of the adhesive tape 113, and the area of the carrier sheet 111 is larger than the area of the release sheet 112. In the adhesive tape 113, the size of the substrate 113A is equal to the size of the adhesive layer 113B. That is, the area of the substrate 113A is equal to the area of the adhesive layer 113B, and the shape of the substrate 113A is the same as the shape of the adhesive layer 113B. The release sheet 112 and the adhesive tape 113 may be configured such that the area of the release sheet 112 is larger than the area of the adhesive tape 113 and the adhesive tape 113 is located within the release sheet 112. That is, the size of the release sheet 112 may be equal to or larger than the size of the adhesive tape 113.

The carrier sheet 111 includes a slit 111A. The slit 111A extends toward the adhesive tape 113 from a portion of an outer edge 111E of the carrier sheet 111 that does not overlap the adhesive tape 113 and has a length that does not reach an outer edge 113E of the adhesive tape 113 as seen in the view facing the plane on which the carrier sheet 111 extends. That is, the slit 111A of the carrier sheet 111 is shaped to extend from the outer edge 111E toward the inside of the outer edge 111E. The slit 111A extends through the carrier sheet 111 in the thickness direction of the carrier sheet 111. In the present embodiment, the slit 111A is a straight I-notch.

In the slit 111A, the end at the outer edge 111E is a basal end, and the end opposite to the basal end is a distal end. The distal end of the slit 111A is located between the outer edge 111E of the carrier sheet 111 and an outer edge 113AE of the substrate 113A as seen in the view facing the plane on which the carrier sheet 111 extends. Thus, since the slit 111A has a length that does not reach the outer edge 113AE of the substrate 113A of the adhesive tape 113, the entire substrate 113A is covered with the carrier sheet 111 until the carrier sheet 111 is peeled off from the substrate 113A. This limits the adhesion of dust or the like on the substrate 113A before the use of the adhesive body 110.

The adhesive tape 113 has a quadrangular shape as seen in the view facing the plane on which the carrier sheet 111 extends. In the example shown in Fig. 21, the adhesive tape 113 has a rectangular shape. Instead, the adhesive tape 113 may have a square shape. The carrier sheet 111 has a rectangular shape in the same manner as the adhesive tape 113. However, the shape of the carrier sheet 111 may be different from the shape of the adhesive tape 113.

The adhesive body 110 satisfies the following conditions 2-1 and 2-2 in a tear test using a right angled tear method in accordance with JIS K 7128-3:1998 "Plastics - Film and sheeting - Determination of tear resistance - Part 3: Right angled tear method."

(Condition 2-1) The displacement amount (mm) of the adhesive tape 113 at break is 3.0 times or more than the displacement amount (mm) of the carrier sheet 111 at break.

(Condition 2-2) The integral value of a tear load applied to the carrier sheet 111 until break is less than or equal to 40 N·mm.

In the condition 2-1, the displacement amount when the adhesive tape 113 breaks is a value obtained by a tear test conducted solely on the adhesive tape 113. In addition, in the conditions 2-1 and 2-2, the displacement amount of the carrier sheet 111 at break and the integral value of the tear load applied to the carrier sheet 111 until the carrier sheet 111 breaks are values obtained by the tear test conducted solely on the carrier sheet 111. The integral value of the tear load applied to the carrier sheet 111 is the area of a region surrounded by the following graph. It is indicated that the relationship between the displacement amount on the horizontal axis and the tear load on the vertical axis is shown in a graph of the tear load applied to the carrier sheet 111 from the start of the tear test to the breakage of the carrier sheet 111.

In the adhesive body 110 of the present disclosure, the displacement amount of the adhesive tape 113 at break is 3.0 times or more than the displacement amount of the carrier sheet 111 at break. Thus, even when a force stretching the carrier sheet 111 acts on the carrier sheet 111 so that it is stretched to such an extent that the carrier sheet 111 breaks, the adhesive tape 113 is less likely to break. Further, the integral value of a tear load applied to the carrier sheet 111 until break is less than or equal to 40 N·mm. Thus, an excessively large force is not required until the carrier sheet 111 breaks after the application of load to the carrier sheet 111 is started. Accordingly, the carrier sheet 111 can be easily peeled off from the adhesive tape 113 by breaking the carrier sheet 111.

To limit situations in which the adhesive tape 113 is excessively stretched when a force is applied to stretch the carrier sheet 111, it is preferable that the displacement amount of the adhesive tape 113 at break is 30.0 times or less the displacement amount of the carrier sheet 111 at break.

The integral value of the tear load applied to the carrier sheet 111 is smaller than the integral value of the tear load applied to the adhesive tape 113. Thus, when a force is applied to break the carrier sheet 111, the carrier sheet 111 breaks without causing the adhesive tape 113 to break. The integral value of the tear load applied to the adhesive tape 113 is the area of a region surrounded by the following graph. It is indicated that the relationship between the displacement amount on the horizontal axis and the tear load on the vertical axis is shown in a graph of the tear load applied to the adhesive tape 113 from the start of the tear test to the breakage of the adhesive tape 113.

Preferably, the adhesive body 10 further satisfies the following conditions 2-3 and 2-4.

(Condition 2-3) The maximum tear load (N) of the carrier sheet 111 is less than or equal to 20 N.

(Condition 2-4) The displacement amount (mm) of the carrier sheet 111 at break is less than or equal to 12 mm.

The maximum tear load applied to the carrier sheet 111 until it breaks 111 is less than or equal to 20 N. This limits situations in which the tear load applied to the carrier sheet 111 at a predetermined moment during a period in which the carrier sheet 111 is stretched becomes excessively large. The maximum tear load on the carrier sheet 111 is larger than the maximum tear load on the adhesive tape 113. In addition, the displacement amount (mm) of the carrier sheet 111 at break is less than or equal to 12 mm. This limits situations in which the amount by which the carrier sheet 111 is stretched at break becomes excessively large. Accordingly, the carrier sheet 111 can be more easily peeled off from the adhesive tape 113 by breaking the carrier sheet 111.

The adhesive body 110 may satisfy the following condition 2-5.

(Condition 2-5) In the tear test using the right angled tear method, the integral value of the tear resistance (N/mm) of the carrier sheet 111 until it breaks is less than or equal to 1000 N.

When the adhesive body 110 satisfies the condition 2-5, the integral value of the tear resistance of the carrier sheet 111 until break is less than or equal to 1000 N. Thus, an excessively large force is not required until the carrier sheet 111 breaks after the application of load to the carrier sheet 111 is started. Accordingly, the carrier sheet 111 can be easily peeled off from the adhesive tape 113 by breaking the carrier sheet 111.

When the adhesive body 110 satisfies the condition 2-5, it is preferable that the adhesive body 110 satisfies the following conditions 2-6 and 2-7.

(Condition 2-6) The tear resistance (N/mm) of the carrier sheet 111 is less than or equal to 376 N/mm.

(Condition 2-7) The displacement amount (mm) of the carrier sheet 111 at break is less than or equal to 12 mm.

When the adhesive body 110 satisfies condition 2-6, it limits situations in which the tear resistance of the carrier sheet 111 at a predetermined moment during a period in which the carrier sheet 111 is stretched becomes excessively large. In addition, when the adhesive body 110 satisfies condition 2-7, it limits situations in which the amount by which the carrier sheet 111 is stretched at break becomes excessively large. Accordingly, the carrier sheet 111 can be more easily peeled off from the adhesive tape 113 by breaking the carrier sheet 111.

Further, the adhesive body 110 may satisfy at least one of the following conditions 2-8 and 2-9. That is, the adhesive body 110 may satisfy only one of the conditions 2-8 and 2-9, or may satisfy both of the conditions 2-8 and 2-9.

(Condition 2-8) In the tear test conducted using a right angled tear method, the displacement amount (mm) of the carrier sheet 111 when the tear load applied to the carrier sheet 111 is 5 N is greater than or equal to 1.5 mm.

(Condition 2-9) In the tear test conducted using a right angled tear method, the tear load applied to the carrier sheet 111 when its displacement amount is 1 mm is less than or equal to 3.5 N.

When the adhesive body 110 satisfies the condition 2-8, the application of a force to the carrier sheet 111 can stretch the carrier sheet 111 to the extent that it is easily torn.

In a case where the adhesive body 110 satisfies condition 2-9, the tear load when the displacement amount is 1 mm is less than or equal to 3.5 N. Thus, only a relatively small force is required to stretch the carrier sheet 111 to the extent that it is easily torn.

The carrier sheet 111 includes a layer formed from a synthetic resin. The carrier sheet 111 includes, for example, a substrate sheet and a separation layer. The separation layer is laminated on the substrate sheet. When the carrier sheet 111 has the substrate sheet and the separation layer, the separation layer is in contact with the substrate 113A of the adhesive tape 113. The substrate sheet may be formed from, for example, a polypropylene (PP) resin, a polyester resin like a polyethylene terephthalate (PET) resin, or a polyvinylidene fluoride (PVDF) resin. The substrate sheet may be any one of a uniaxially stretched sheet, a biaxially stretched sheet, or an unstretched sheet. The separation layer may be formed from, for example, a silicone resin.

The carrier sheet 111 may include only the substrate sheet described above. In this case, a contact surface of the substrate sheet that is in contact with the substrate 113A may be subject to processing for facilitating peeling of the substrate 113A from the substrate sheet. The processing on the contact surface of the substrate sheet may be, for example, embossing.

The carrier sheet 111 may include a paper layer in addition to the layer formed from a synthetic resin. In this case, the carrier sheet 111 includes a paper substrate layer and a synthetic resin layer laminated on the substrate layer. The synthetic resin layer may be formed from, for example, a PP resin. The thickness of the carrier sheet 111 may be, for example, between 30 $\mu$m and 400 $\mu$m, inclusive.

The release sheet 112 is configured such that the peel strength between the release sheet 112 and the adhesive layer 113B is smaller than the peel strength between the carrier sheet 111 and the substrate 113A. The release sheet 112 is formed from a synthetic resin. The release sheet 112 includes, for example, a substrate sheet and a separation layer. The separation layer is laminated on the substrate sheet. When the release sheet 112 includes a substrate sheet and a separation layer, the separation layer is in contact with the adhesive layer 113B of the adhesive tape 113. The substrate sheet may be formed from, for example, a polyethylene terephthalate (PET) resin. The substrate sheet may be any one of a uniaxially stretched sheet, a biaxially stretched sheet, or an unstretched sheet. The separation layer may be formed from, for example, a silicone resin.

The release sheet 112 may include only the substrate sheet described above. In this case, a contact surface of the substrate sheet that is in contact with the adhesive layer 113B may be subject to processing for facilitating peeling of the adhesive layer 113B from the substrate sheet. The processing on the contact surface of the substrate sheet may be, for example, embossing. The thickness of the release sheet 112 may be, for example, between 12 $\mu$m and 350 $\mu$m, inclusive.

The substrate 113A is formed from a synthetic resin. The synthetic resin used to form the substrate 113A may be, for example, a polyurethane resin. This provides the substrate 113A with a relatively high adhesive suitability. The thickness of the substrate 113A may be, for example, between 5 $\mu$m and 30 $\mu$m, inclusive. The substrate 113A that is formed from a polyurethane resin and is relatively thin extends well even if the external force applied to the substrate 113A for stretching the substrate 113A is relatively small. Thus, the substrate 113A has a relatively high conformity to the shape of the target to which the adhesive tape 113 will be adhered and can achieve high adhesion to the adhesion target.

The substrate 113A, which has a relatively high conformity to the shape of the adhesion target, also has a relatively high conformity to the carrier sheet 111, which covers the substrate 113A. Thus, when a force is applied to the carrier sheet 111 to peel it off from the substrate 113A, the substrate 113A easily deforms following the deformation of the carrier sheet 111.

The tensile elongation at break of the substrate 113A may be, for example, greater than or equal to 130%. The tensile elongation at break can be obtained in accordance with JIS K 7161-1:2014 (ISO 527-1) "Plastics - Determination of tensile properties - Part 1: General principles" and JIS K 7127:1999 (ISO 527-3) "Plastics - Determination of tensile properties - Part 3: Test conditions for films and sheets." If the measurement object does not have a yield point, the tensile strain at break is measured. If it has a yield point, the nominal tensile strain at break is measured. The tensile elongation at break can be determined using these measurement values.

The tensile strength of the substrate 113A at 100% elongation may be, for example, less than or equal to 4 N/cm. The tensile strength at 100% elongation is a value obtained by dividing, by the width of the test piece, the magnitude of the force measured when the strain defined in JIS K 7161 - 1:2014 (ISO 527-1) "Plastics - Determination of tensile properties - Part 1: General principles" reaches a specified value (100%). The tensile strength at 100% elongation (T) (N/cm) can be determined by the following equation.

$$T = F/W$$

In this equation, F is the magnitude of the measured force (N) and W is the width of the test piece (cm).

The substrate 113A may be formed from synthetic resins other than a polyurethane resin. Examples of synthetic resins other than a polyurethane resin include a polyvinylidene fluoride resin, an ethylene-vinyl acetate copolymer resin, a polypropylene resin, and a polyethylene terephthalate resin.

In the same manner as the substrate 113A, the adhesive layer 113B is formed from a synthetic resin. The synthetic resin used to form the adhesive layer 113B may be, for example, a polyurethane resin. The thickness of the adhesive layer 113B may be, for example, between 5 μm and 25 μm, inclusive.

At least one of the substrate 113A and the adhesive layer 113B may contain an active ingredient for skin. That is, only the substrate 113A may contain an active ingredient, only the adhesive layer 113B may contain an active ingredient, or both the substrate 113A and the adhesive layer 113B may contain an active ingredient. The active ingredient may be, for example, a cosmetic ingredient, a beauty ingredient, or a pharmaceutical ingredient.

In the adhesive body 110, the substrate 113A may include a polyurethane resin, and the carrier sheet 111 may include a polypropylene resin or a polyvinylidene fluoride resin. In this case, the substrate 113A includes a polyurethane resin, and the carrier sheet 111 includes a polypropylene resin or a polyvinylidene fluoride resin. This provides the adhesive body 110 which has an adhesive tape 13 that easily stretches with a relatively small force and in which the displacement amount of the carrier sheet 111 at break is less likely to be excessively large.

Operation

First, the user of the adhesive body 110 peels off the release sheet 112 from the adhesive layer 113B of the adhesive tape 113. Next, the user adheres the adhesive layer 113B to the target to which the adhesive tape 113 should be adhered. The adhesion target of the adhesive tape 113 is, for example, human skin, as described above. Then, the user peels off the carrier sheet 111 from the substrate 113A of the adhesive tape 113.

When the adhesive tape 113 is adhered along a relatively flat surface of the skin of a human, for example, the palm of the hand, since the carrier sheet 111 has the slit 111A, the user holds a portion of the carrier sheet 111 defining the slit 111A between the thumb and the index finger using the slit 111A of the carrier sheet 111 as a mark. Next, the user applies force to the carrier sheet 111 in the direction in which the slit 111A extends, as seen in the view facing the plane on which the carrier sheet 111 extends.

Further, the adhesive tape 113 may be adhered to a human finger. In this case, after the adhesive tape 113 is placed on the finger, the carrier sheet 111 is pulled together with the adhesive tape 113. As a result, the carrier sheet 111 is torn from the slit 111A. Even when the adhesive tape 113 is adhered to the finger, the carrier sheet 111 may be torn by the same method as in the case of adhering the adhesive tape 113 along a flat surface. That is, after the adhesive tape 113 is placed on the finger, a portion defining the slit 111A is held between the thumb and the index finger. Next, the user applies force to the carrier sheet 111 in the direction in which the slit 111A extends, as seen in the view facing the plane on which the carrier sheet 111 extends. In any case, the force in the direction in which the slit 111A extends can be applied to the carrier sheet 111.

The above-described adhesive body 110 of the present disclosure satisfies the conditions 2-1 and 2-2. This limits breakage of the adhesive tape 113. Further, an excessively large force is not required until the carrier sheet 111 breaks after the application of load to the carrier sheet 111 is started. Accordingly, the carrier sheet 111 can be easily peeled off from the adhesive tape 113 by breaking the carrier sheet 111.

Test Examples

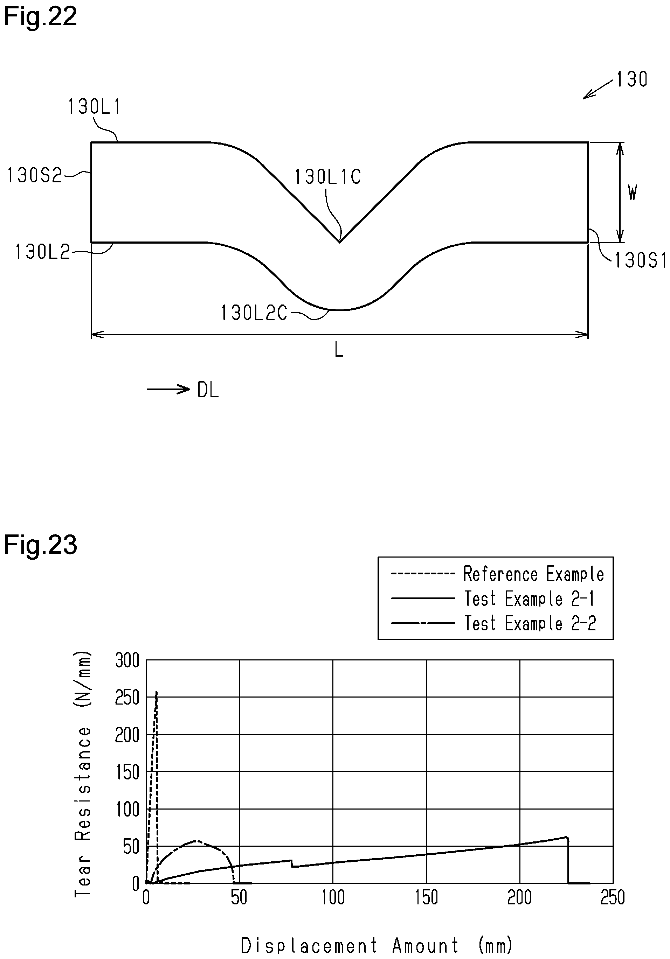

Test examples will now be described with reference to Figs. 22 to 26 and Tables 2, 3, and 4.

Test Example 2-1

A biaxially oriented polypropylene (OPP) film (FOR-MP, 40 μm thick, manufactured by Futamura Chemical Co., Ltd.) with one of two opposing surfaces being a matte surface was prepared as a carrier sheet. Then, an aqueous polyurethane (TAKELAC® WS-6021, manufactured by Mitsui Chemicals, Inc.) containing ether-based polyol was coated onto the matte surface of the OPP film. After that, a precursor layer was formed by drying the aqueous polyurethane. Then, the precursor layer was aged at room temperature to obtain a substrate having a thickness of 15 μm.

A separation film (Cerapeel® WZ, 75 μm thick, manufactured by TORAY ADVANCED FILM Co., Ltd) composed of a PET film and a separation layer that was formed from a silicone resin was prepared as a release sheet. Next, a urethane-based adhesive (SP-205, manufactured by Toyochem Co., Ltd.) as the main agent and a curing agent (T-501B, manufactured by Toyochem Co., Ltd.) were prepared. After adding the curing agent to the main agent, the main agent and the curing agent were stirred to obtain a coating solution for forming the adhesive layer. The coating solution was applied to the separation film, and then the solution was dried to obtain an adhesive layer with a thickness of 15 μm.

Then, the substrate and the adhesive layer were bonded to each other to obtain a laminate in which the OPP film, the adhesive tape, and the separation film were laminated in this order. Next, half cutting was performed on the laminate to cut out a rectangular area that extends through only the separation film and the adhesive tape. Subsequently, a portion of each of the separation film and the adhesive tape outside the section subjected to the half cutting was removed from the OPP film. Thus, the size of the separation film and the size of the adhesive tape were made smaller than the size of the OPP film. Then, a straight slit was formed in a portion of the OPP film located outside the portion where the adhesive tape was laminated. Consequently, the adhesive body of test example 2-1 was obtained.

Test Example 2-2

The adhesive body of test example 2-2 was obtained using the same method as in test example 2-1, except that the aqueous polyurethane used in the adhesive body of test example 2-1 was changed to an aqueous polyurethane (TAKELAC W-6020, manufactured by Mitsui Chemicals) containing ether-based polyol with a glass transition temperature that is higher than that of the aqueous polyurethane of test example 1.

Test Example 2-3

The adhesive body of test example 2-3 was obtained using the same method as in test example 2-1, except that the OPP film in the adhesive body of test example 2-1 was changed to a polyethylene terephthalate (PET) film (S10, 50 $\mu$m thick, manufactured by Toray Industries, Inc.).

Test Example 2-4

The adhesive body of test example 2-4 was obtained using the same method as in test example 2-1, except that the aqueous polyurethane used in the adhesive body of test example 2-1 was changed to an aqueous polyurethane (TAKELAC W-6020, manufactured by Mitsui Chemicals) containing ether-based polyol with a glass transition temperature that is higher than that of the aqueous polyurethane of test example 1 and the OPP film was changed to a polyethylene terephthalate (PET) film (S10, 75 $\mu$m thick, manufactured by Toray Industries, Inc.). Other than that, the adhesive body of test example 2-4 was obtained using the same method as that of test example 2-1.

Test Example 2-5

The adhesive body of test example 2-5 was obtained using the same method as in test example 2-1, except that the aqueous polyurethane used in the adhesive body of test example 2-1 was changed to an aqueous polyurethane (TAKELAC WPB-341, manufactured by Mitsui Chemicals) with a glass transition temperature that is higher than that of the aqueous polyurethane of test example 1.

Evaluation Method

Right Angled Tear Method

The right angled tear method will now be described with reference to Fig. 22.

The displacement amount and tear resistance of the carrier sheet and the displacement amount of the adhesive tape were measured using a method in accordance with JIS K 7128-3:1998 "Plastics - Film and sheeting - Determination of tear resistance - Part 3: Right angled tear method."

To evaluate the carrier sheet, a test piece having a shape shown in Fig. 22 was cut out from the carrier sheet of each test example. To evaluate the adhesive tape, a test piece having a shape shown in Fig. 22 was cut out from the adhesive tape of each test example. The shape of the test piece shown in Fig. 22 is a shape defined in JIS K 7128 3:1998, which has been described above.

As shown in Fig. 22, the test piece 130 has a substantially V-shape. The test piece 130 includes a first long side 130L1 having a substantially V-shape and a second long side 130L2 facing the first long side 130L1 and having a substantially V-shape. In the direction in which each long side 130L1, 130L2 extends, a line segment connecting first ends of the long sides 130L1, 130L2 is a first short side 130S1. In the direction in which each long side 130L1, 130L2 extends, a line segment connecting second ends of the long sides 130L1, 130L2 is a second short side 130S2. Each short side 130S1, 130S2 has a straight shape. Length L of the test piece 130 is measured from the first short side 130S1 to the second short side 130S2. Width W of the test piece 130 is the length of each short side 130S1, 130S2. Length L is set to 100 mm, and width W is set to 20 $\pm$ 1 mm. The first long side 130L1 is bent at a right angle at a midpoint 130L1C in the direction in which the first long side 130L1 extends. The second long side 130L2 is curved at a midpoint 130L2C in the direction in which the second long side 130L2 extends. To create the test piece 130 from the adhesive body of each test example, the test piece 130 was cut out from the adhesive body such that the test piece 130 was torn in a direction that is orthogonal to a longitudinal direction DL during the manufacturing of the adhesive body.

Using a tensile testing machine (Autograph® AGS-X, load cell 1kN, manufactured by Shimadzu Corporation), the first short side 130S1 and the second short side 130S2 were pulled in opposite directions. The tearing speed was set to 500 mm/min. In addition, all tear loads applied to the test piece during the test were recorded over time, and the displacement amount of the test piece 130 during the test was recorded over time. The displacement amount of the test piece 130 refers to the length by which the test piece 130 is stretched in the direction it is pulled, from the start of the test (with an initial displacement amount set to 0 mm) until the test piece 130 breaks due to the applied tear load.

Five test pieces were prepared for each test example, and each numerical value described below was measured or calculated for each test piece. Then, the average value of the numerical values of the five test pieces was calculated, and the average value was set as the numerical value of each test example.

Adhesive Tape

All tear loads applied to the test piece formed from the adhesive tape included in each adhesive body were recorded over time, and the displacement amount of the test piece was recorded over time. Then, by dividing the tear load (N) by the thickness (mm) of the test piece, the tear resistance (N/mm) was calculated for all the tear loads applied to the test piece. Further, from the displacement amount and the tear resistance, a graph showing the relationship between the displacement amount and the tear resistance was created.

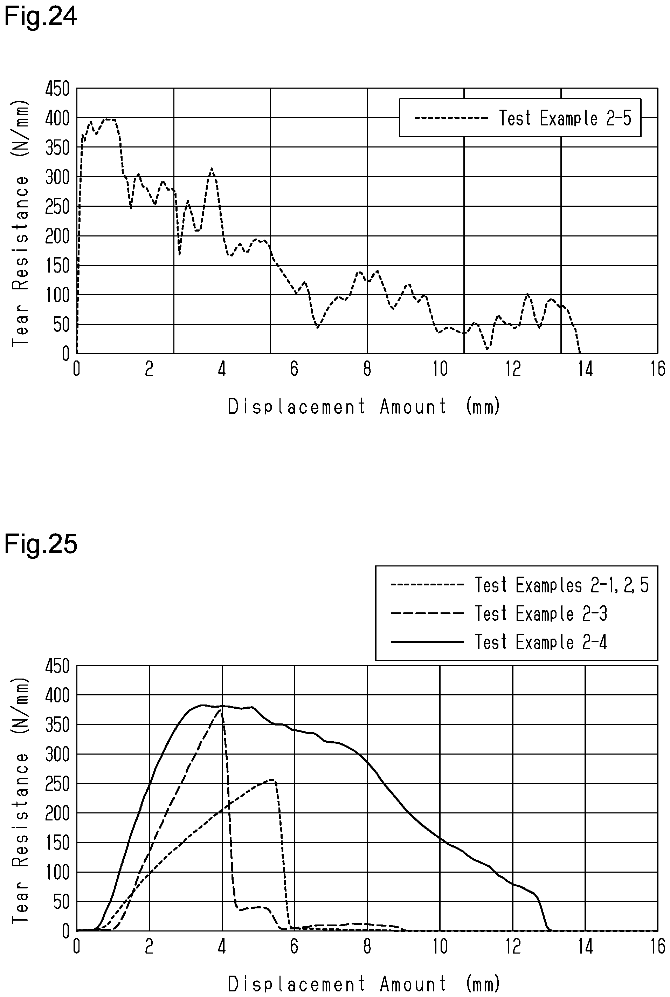

Fig. 23 and Fig. 24 are examples of graphs each showing the relationship between the displacement amount and the tear resistance obtained for the test pieces. In Fig. 23, the graph obtained for the test piece of test example 2-1 is indicated by the solid line, and the graph obtained for the test piece of test example 2-2 is indicated by the alternate long and short dashed line. Since the adhesive tape, which is the test piece of test example 2-3, was the same as the adhesive tape of test example 2-1, a graph similar to that of the test piece of test example 2-1 was also obtained for the test piece of test example 2-3. Since the adhesive tape, which is the test piece of test example 2-4. was the same as the adhesive tape of test example 2-2, a graph similar to that of the test piece of test example 2-1 was also obtained for the test piece of test example 2-4. In addition, in Fig. 23, a graph obtained for the carrier sheet of test example 2-1, which is an example of the carrier sheet, is shown by the broken line as a reference example. In Fig. 24, a graph obtained for the test piece of test example 2-5 is indicated by the broken line.

For each of the graphs shown in Figs. 23 and 24, the displacement amount at the point in time when the tear resistance became 0 for the first time after the application of the tear load to the test piece was read as the displacement amount at the point in time the test piece broke.

Carrier Sheet

As described above, all tear loads applied to the test piece formed from the carrier sheet included in each adhesive body were recorded over time, and the displacement amount of the test piece was recorded over time. Then, by dividing the tear load (N) by the thickness (mm) of the test piece, the tear resistance (N/mm) was calculated for all the tear loads applied to the test piece. Further, from the displacement amount and the tear resistance, a graph showing the relationship between the displacement amount and the tear resistance was created.

Fig. 25 is an example of a graph showing the relationship between the displacement amount and the tear resistance obtained for the test piece. In Fig. 25 shows a graph obtained for the test pieces of test examples 2-1 to 2-5.

For each graph shown in Fig. 25, the maximum value of the graph was read as the maximum value of the tear resistance. In JIS K 7128 3:1998, the maximum value in each graph shown in Fig. 6 is defined as the tear resistance. That is, in JIS K 7128 3:1998, the value obtained by dividing the maximum tear load by the thickness is defined as the tear resistance. However, for the purposes of calculating the integral value of tear resistance in the present disclosure, all the applied tear loads on the test pieces are calculated as values obtained by dividing the tear loads by the thicknesses. Therefore, in the present disclosure, the tear resistance defined in JIS K 7128-3:1998 is referred to as the maximum tear resistance, and the value obtained by dividing each tear load by the thickness is referred to as the tear resistance.

For each test piece, the integral value (N) of the tear resistance during the period from the start of the application of the tear load to the carrier sheet to the breakage of the carrier sheet was calculated. The integral value (N) of the tear resistance is equal to the area of the region surrounded by each graph shown in Fig. 25 and the horizontal axis.

For each graph shown in Fig. 25, the displacement amount at the point in time when the tear resistance became 0 for the first time after the application of the tear load to the test piece was read as the displacement amount at the point in time the test piece broke. Further, in each graph shown in Fig. 25, the tear resistance when the displacement amount of the carrier sheet is 1 mm was read.

Further, from the displacement amount and the tear load, a graph showing the relationship between the displacement amount and the tear load was created.

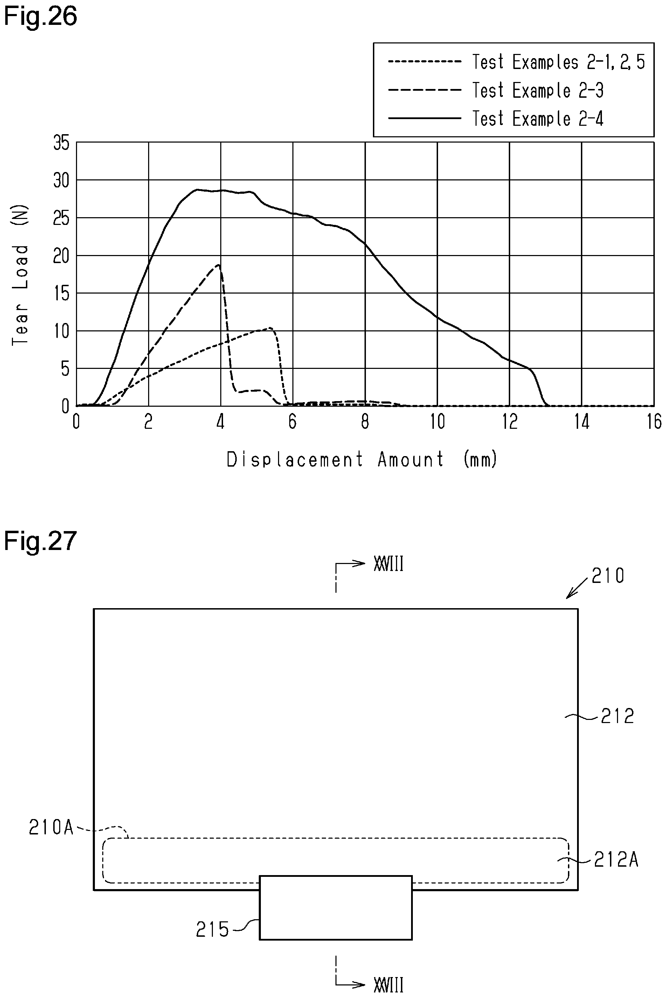

Fig. 26 is an example of a graph showing the relationship between the displacement amount and the tear load obtained for the test piece. Fig. 26 shows a graph obtained for the test pieces of test examples 2-1 to 2-5.

For each test piece, the integral value (N·mm) of the tear load during the period from the start of the application of the tear load to the carrier sheet to the breakage of the carrier sheet was calculated. The integral value (N·mm) of the tear load is equal to the area of the region surrounded by each graph and the horizontal axis shown in Fig. 26.

For each graph shown in Fig. 26, the maximum value of the graph was read as the maximum tear load. From each graph shown in Fig. 26, the displacement amount of the test piece when the tear load was 5 N was read. In addition, from each graph shown in Fig. 26, the tear load when the displacement amount was 1 mm was read.

For each graph shown in Fig. 26, the displacement amount at the time when the tear load became 0 for the first time after the application of the tear load to the test piece was read as the displacement amount at the time of breakage of the test piece. The displacement amount at break that is read from each graph shown in Fig. 26 is the same as the displacement amount at break that is read from the graph obtained for the same test piece in the graph shown in Fig. 25.

Tear Evaluation of Carrier Sheet and Adhesive Tape

In each test example, the size of the adhesive tape was set to 10 cm square, and the size of the carrier sheet was set to 12 cm square. Then, only one straight slit having a length of 1 cm from the outer edge of the carrier sheet was formed in the carrier sheet. Next, after the adhesive tape of each test example was adhered to the skin, the carrier sheet was peeled off from the adhesive tape. For each test example, five adhesive bodies were evaluated.

The tear performance of the carrier sheet was evaluated in the following three grades.

◎ (excellent) The resistance felt when tearing the carrier sheet is almost negligible.
o The resistance felt when tearing the carrier sheet is relatively small.
△ The resistance felt when tearing the carrier sheet is relatively large.

The tear performance of the adhesive tape was evaluated in the following two grades.

o When the carrier sheet is peeled off from the adhesive tape, the adhesive tape is not torn.
× (unacceptable) When the carrier sheet is peeled off from the adhesive tape, the adhesive tape is also torn.

Evaluation Results

The adhesive body of each test example will now be described with reference to Tables 2, 3, and 4.

The test pieces of the test examples were evaluated using the above-described right angled tear method. The results were as shown in Tables 2 and 3 below. In addition, the results of evaluating the release performance of the carrier sheet with the above-described adhesive body applied to the skin were as shown in Table 3.

Table 2

| | Adhesive Tape Displacement Amount (mm) | Carrier Sheet Displacement Amount (mm) | Ratio of Displacement Amounts |
|---|---|---|---|
| Test Example 2-1 | 232.9 | 8.5 | 27.4 |
| Test Example 2-2 | 46.8 | 8.7 | 5.4 |
| Test Example 2-3 | 227.7 | 9.0 | 25.3 |
| Test Example 2-4 | 48.6 | 13.5 | 3.6 |
| Test Example 2-5 | 10.3 | 8.5 | 1.2 |

As shown in Table 2, for each of the adhesive tapes of test examples 2-1 and 2-3, it was observed that the displacement amount at break of the adhesive tape was included in the range of between 227. 7 mm and 232. 9 mm, inclusive. For the adhesive tape of each of test examples 2-2 and 2-4, it was observed that the displacement amount at break of the adhesive tape was included in the range of between 46.8 mm and 48.6 mm, inclusive. For the adhesive tape of test example 2-5, it was observed that the displacement amount at break of the adhesive tape was 10.3 mm. Thus, it was observed that the displacement amount of the adhesive tape with a substrate formed from a urethane resin with a relatively low glass transition temperature was greater than the displacement amount of the adhesive tape with a substrate formed from a urethane resin with a relatively high glass transition temperature.

For each of the carrier sheets of test examples 2-1, 2-2, and 2-5, it was observed that the displacement amount at break of the carrier sheet was included in the range of between 8.5 mm and 8.7 mm, inclusive. For each of the carrier sheets of test examples 2-3 and 2-4, it was observed that the displacement amount at break of the carrier sheet was included in the range of between 9.0 mm and 13.5 mm, inclusive and the thinner the carrier sheet, the smaller the displacement amount of the carrier sheet.

Thus, for test examples 2-1 to 2-4, it was observed that the ratio obtained by dividing the displacement amount of the adhesive tape by the displacement amount of the carrier sheet was included in the range of between 3.6 and 27.4, inclusive. For test example 2-5, it was observed that the ratio obtained by dividing the displacement amount of the adhesive tape by the displacement amount of the carrier sheet was 1.2.

The carrier sheet was evaluated using the right angled tear method. The results were as shown in Table 3 below.

Table 3

| | Tear Load Integral Value (N•mm) | Maximum Tear Load (N) | Tear Resistance Integral Value (N) | Maximum Tear Resistance (N/mm) | Displacement Amount at Application of 5N (mm) | Tear Load at Displacement of 1mm (N) | Tear Resistance at Displacement of 1mm (N/mm) |
|---|---|---|---|---|---|---|---|
| Test Example 2-1 | 28.2 | 9.9 | 708 | 246.9 | 2.9 | 1.0 | 25.0 |
| Test Example 2-2 | 28.2 | 9.9 | 708 | 246.9 | 2.9 | 1.0 | 25.0 |
| Test Example 2-3 | 37.2 | 18.7 | 743 | 375.9 | 1.8 | 3.4 | 67.3 |
| Test Example 2-4 | 222.9 | 28.5 | 2972 | 379.9 | 1.0 | 5.2 | 68.7 |
| Test Example 2-5 | 28.2 | 9.9 | 708 | 246.9 | 2.9 | 1.0 | 25.0 |

As shown in Table 3, it was observed that the integral value of the tear load was 28.2 N·mm in test examples 2-1 and 2-2, 37.2 N·mm in test example 2-3, and 222.9 N·mm in test example 2-4. It was observed that the integral value of the tear load was 28.2 N·mm in test example 2-5.

It was observed that the maximum tear load was 9.9 N in test examples 2-1 and 2-2, 18.7 N in test example 2-3, and 28.5 N in test example 2-4. It was observed that the maximum tear load was 9.9 N in test example 2-5.

It was observed that the integral value of the tear resistance was 708 N in test examples 2-1 and 2-2, 743 N in test example 2-3, and 2972 N in test example 2-4. It was observed that the integral value of tear resistance was 708 N in test example 2-5.

It was observed that the maximum tear resistance was 246.9 N/mm in test examples 2-1 and 2-2, 375.9 N/mm in test example 2-3, and 379.9 N/mm in test example 2-4. It was observed that the maximum tear resistance was 246.9 N/mm in test example 2-5.

It was observed that the displacement amount when a tear load of 5 N was applied to the test piece 130 was 2.9 mm in test examples 2-1 and 2-2, 1.8 mm in test example 2-3, and 1. 0 mm in test example 2-4. It was observed that the displacement amount when a tear load of 5 N was applied to the test piece 130 was 2.9 mm in test example 2-5.

It was observed that the tear load when the displacement amount of the test piece 130 was 1 mm was 1.0 N in test examples 2-1 and 2-2, 3.4 N in test example 2-3, and 5.2 N in test example 2-4. It was observed that the tear load when the displacement amount of the test piece 130 was 1 mm was 1.0 N in test example 2-5.

It was observed that the tear resistance when the displacement amount of the test piece 130 was 1 mm was 25.0 n/mm in test examples 2-1 and 2-2, 67.3 N/mm in test example 2-3, and 68.7 N/mm in test example 2-4. It was observed that the tear resistance when the displacement amount of the test piece 130 was 1 mm was 25.0 N/mm in test example 2-5.

The evaluation results of the tear performance of the carrier sheet and the tear performance of the adhesive tape were as shown in Table 4 below.

Table 4

| | Carrier Sheet Tear Evaluation | Adhesive Tape Tear Evaluat on |
|---|---|---|
| Test Example 2-1 | ◎ | ○ |
| Test Example 2-2 | ◎ | ○ |
| Test Examp.e 2-3 | ○ | ○ |
| Test Example 2-4 | Δ | ○ |
| Test Example 2-5 | ◎ | × |

In the evaluation of the tear performance of the carrier sheet, it was observed that in the adhesive bodies of test examples 2-1, 2-2, and 2-5, the resistance felt when tearing the carrier sheet was almost negligible. In test examples 2-1, 2-2, and 2-5, it was observed that the carrier sheet was torn along the slit when the carrier sheet was peeled off from the adhesive tape and thus the carrier sheet was split into two. Test examples 2-1, 2-2, and 2-5 indicate that since both the maximum tear load and the displacement amount are reduced to a relatively low level, the integral value of the tear load is the smallest and thus the resistance felt when breaking the carrier sheet is the least noticeable.

In addition, in the adhesive body of test example 2-3, it was observed that the resistance when peeling off the carrier sheet was relatively small. In test example 2-3, it was observed that the carrier sheet was torn along the slit when the carrier sheet was peeled off from the adhesive tape and thus the carrier sheet was split into two. In the carrier sheet of test example 2-3, compared with the carrier sheets of test examples 2-1 and 2-2, the integrated value of the tear load, the maximum tear load, and the displacement amount until break are all relatively large. This indicates that the resistance when peeling off the carrier sheet of test example 2-3 is easily felt as compared with the case of peeling off the carrier sheets of test examples 2-1 and 2-2.

In the adhesive body of test example 2-4, it was observed that the resistance when peeling off the carrier sheet was relatively large. In test example 2-4, it was observed that the carrier sheet was torn along the slit when the carrier sheet was peeled off from the adhesive tape and thus the carrier sheet was split into two. However, in test example 2-4, it was observed that the tearing path of the carrier sheet had irregularities compared with the adhesive bodies in test examples 2-1 to 2-3. In the carrier sheet of test example 2-4, compared with the carrier sheets of test examples 2-1 and 2-2, the integral value of the tear load and the maximum tear load are significantly large, and the displacement amount when breaking the carrier sheet is also relatively large. This indicates that the resistance when peeling off the carrier sheet is relatively large.

In the evaluation of the tear performance of the adhesive tape, it was observed that the adhesive tapes were not torn when the carrier sheets were peeled off in the adhesive bodies of test examples 2-1 to 2-4. It was observed that the adhesive tape was torn when the carrier sheet was peeled off from the adhesive body of test example 2-5. These results indicate that when the displacement amount of the adhesive tape at break is 3.0 times or more than the displacement amount of the carrier sheet at break, the tearing of the adhesive tape during the peeling of the carrier sheet is limited.

As described above, the adhesive body of the second embodiment provides the following advantages.

(2-1) Since the adhesive body 110 satisfies the condition 2-1, the adhesive tape 113 is less likely to break even when the carrier sheet 111 is stretched to such an extent that the carrier sheet 111 breaks. Further, since the adhesive body 110 satisfies the condition 2-2, an excessively large force is not required until the carrier sheet 111 breaks after the application of load to the carrier sheet 111 is started.

(2-2) When the adhesive body 110 satisfies the condition 2-3, it limits situations in which the tear load applied to the carrier sheet 111 at a predetermined moment during a period in which the carrier sheet 111 is stretched becomes excessively large. In addition, when the adhesive body 110 satisfies the condition 2-4, it limits situations in which the amount by which the carrier sheet 111 is stretched at break becomes excessively large.

(2-3) When the adhesive body 110 satisfies the condition 2-5, an excessively large force is not required until the carrier sheet 111 breaks after the application of load to the carrier sheet 111 is started.

(2-4) When the adhesive body 110 satisfies the condition 2-6, it limits situations in which the tear resistance of the carrier sheet 111 at a predetermined moment during a period in which the carrier sheet 111 is stretched becomes excessively large. In addition, when the adhesive body 110 satisfies the condition 2-7, it limits situations in which the amount by which the carrier sheet 111 is stretched at break becomes excessively large. Accordingly, the carrier sheet 111 can be more easily peeled off from the adhesive tape 113 by breaking the carrier sheet 111.

(2-5) When the adhesive body 110 satisfies the condition 2-8, the application of a force to the carrier sheet 111 can stretch the carrier sheet 111 to the extent that it is easily torn.

(2-6) In a case where the adhesive body 110 satisfies condition 2-9, the tear load when the displacement amount is 1 mm is less than or equal to 3.5 N. Thus, only a relatively small force is required to stretch the carrier sheet 111 to such an extent that it is easily torn.

(2-7) When the substrate 113A includes a polyurethane resin and the carrier sheet 111 includes any one of a polypropylene resin, a polyethylene terephthalate resin, and a polyvinylidene fluoride resin, it provides the adhesive body 110 which has an adhesive tape that easily stretches with a relatively small force and in which the displacement amount at break is less likely to be excessively large.

Modifications of Second Embodiment

The above-described second embodiment may be implemented in combination with the modifications of the first embodiment.

Third Embodiment

The adhesive body and the method for manufacturing the adhesive body according to a third embodiment will now be described with reference to Figs. 27 to 35.

Adhesive Body

First Example

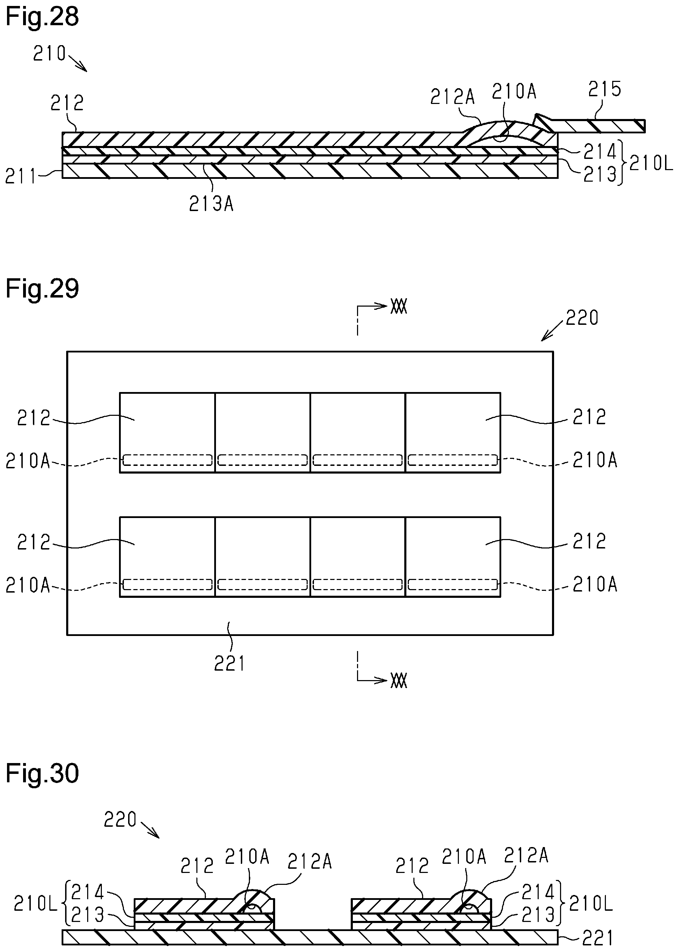

A first example of the adhesive body, which is a skin adhesive sheet, will now be described with reference to Figs. 27 and 28. Fig. 27 shows a structure of the adhesive body as seen in a view facing a plane on which the adhesive body extends.

As shown in Fig. 27, the outer shape of an adhesive body 210 is a rectangular shape as seen in the view facing the plane on which the adhesive body 210 extends. The adhesive body 210 includes layers. One of the layers that serves as an outer surface of the adhesive body 210 is a carrier layer 212.

The adhesive body 210 has a gap 210A extending along one side of the outer shape. The gap 210A extends over the entire side along which the gap 210A extends. The gap 210A may extend along only part of one side.

The adhesive body 210 includes a grip portion 215. The grip portion 215 is attached to part of the carrier layer 212. As seen in a view facing the carrier layer 212, the grip portion 215 overlaps part of the gap 210A and protrudes outward from the outer shape of the adhesive body 210. The grip portion 215 is attached to the carrier layer 212 so as to overlap the center of the gap 210A in the direction in which the gap 210A extends, as seen in the view facing the carrier layer 212. The grip portion 215 has a rectangular shape in the example shown in Fig. 25.

Fig. 28 shows a cross-sectional structure of the adhesive body 210 along line XXVIII-XXVIII shown in Fig. 27.

As shown in Fig. 28, the adhesive body 210 includes a release layer 211, the carrier layer 212, an adhesive layer 213, and a substrate layer 214. The release layer 211 serves as the outer surface of the adhesive body 210. The adhesive layer 213 is located between the release layer 211 and the carrier layer 212, and has an adhesive surface 213A that is in contact with the release layer 211. The substrate layer 214 is located between the adhesive layer 213 and the carrier layer 212, and is in contact with the adhesive layer 213 and the carrier layer 212. The substrate layer 214 has a higher tensile elongation at break than the release layer 211 and the carrier layer 212. In the adhesive body 210, the adhesive surface 213A from which the release layer 211 has been peeled off is adhered to the skin.

The outer shape of a laminate 210L, including the adhesive layer 213 and the substrate layer 214, includes a portion that matches the outer shape of the carrier layer 212. In this example, as described above with reference to Fig. 25, the outer shape of the carrier layer 212 has a rectangular shape. The laminate 210L has a rectangular outer shape, and has the same shape and the same size as the carrier layer 212. Thus, in this example, the outer shape of the laminate 210L includes sides, and all the sides included in the outer shape of the laminate 210L match those of the outer shape of the carrier layer 212.

The adhesive body 210 includes the gap 210A in part of the portion of the outer shape of the laminate 210L that matches the outer shape of the carrier layer 212. The gap 210A of the present embodiment is an example of a gap located in at least part of the outer shape of the laminate 210L, which includes the adhesive layer 213 and the substrate layer 214. In the gap 210A, the substrate layer 214 is separated from the carrier layer 212. In this example, one of the sides included in the laminate 210L is included in the portion that matches the outer shape of the carrier layer 212. The gap 210A has a length over the entirety of that side. Thus, as seen in the view facing the plane on which the adhesive body 210 extends, the gap 210A is long enough to cover the entirety of one side of the rectangle, which is the outer shape of the adhesive body 210.

The adhesion strength between the substrate layer 214 and the carrier layer 212 is less than or equal to 2N/25 mm. The adhesion strength between the substrate layer 214 and the carrier layer 212 is a value measured using a method in accordance with "180° peel strength" of JIS Z 0237:2009 "Testing methods of pressure-sensitive adhesive tapes and sheets."

As described above, in the adhesive body 210, the gap 210A in which the substrate layer 214 is separated from the carrier layer 212 is located at the portion where the adhesion strength between the substrate layer 214 and the carrier layer 212 is less than or equal to 2 N/25 mm and the outer shape of the laminate 210L matches the outer shape of the carrier layer 212. Thus, the substrate layer 214 having a relatively high tensile elongation at break is easily peeled off from the carrier layer 212. In addition, since the gap 210A between the carrier layer 212 and the substrate layer 214 extends along the entirety of one side included in the outer shape of the laminate 210L, the substrate layer 214 is more easily peeled off from the carrier layer 212.

The gap 210A is a closed space. In this example, the carrier layer 212 includes a curved portion 212A along one side included in the outer shape of the carrier layer 212. In the cross-section taken along a plane orthogonal to the thickness direction of the carrier layer 212 and the direction in which the curved portion 212A extends, the curved portion 212A has a substantially arcuate shape protruding in a direction away from the substrate layer 214. Since the edge of the curved portion 212A is bonded to the substrate layer 214, the gap 210A, which is a closed space, is formed. Since the gap 210A is a closed space, the collection of foreign matter on a portion of the substrate layer 214 that is separated from the carrier layer 212 is limited.

The release layer 211 is configured such that the peel strength between the release layer 211 and the adhesive layer 213 is smaller than the peel strength between the carrier layer 212 and the substrate layer 214. The release layer 211 is formed from a synthetic resin. The release layer 211 includes, for example, a substrate sheet and a separation layer. The separation layer is laminated on the substrate sheet. When the release layer 211 includes a substrate sheet and a separation layer, the separation layer is in contact with the adhesive layer 213. The substrate sheet may be formed from, for example, a polyethylene terephthalate resin. The substrate sheet may be any one of a uniaxially stretched sheet, a biaxially stretched sheet, or an unstretched sheet. The separation layer may be formed from, for example, a silicone resin.

The release layer 211 may include only the substrate sheet described above. In this case, a contact surface of the substrate sheet that is in contact with the adhesive surface 213A of the adhesive layer 213 may be subject to processing for facilitating peeling of the adhesive layer 213 from the substrate sheet. The processing on the contact surface of the substrate sheet may be, for example, embossing.

The thickness of the release layer 211 may be, for example, between 12 $\mu$m and 350 $\mu$m, inclusive.

The carrier layer 212 is formed from a synthetic resin. The carrier layer 212 includes, for example, a substrate sheet and a separation layer. The separation layer is laminated on the substrate sheet. When the carrier layer 212 includes a substrate sheet and a separation layer, the separation layer is in contact with the substrate layer 214. The substrate sheet may be formed from, for example, any of a polyolefin resin and a polyester resin. The polyester resin may be, for example, a polyethylene terephthalate resin. The polyolefin resin may be, for example, a polypropylene resin. The substrate sheet is preferably formed from a polyolefin resin. The substrate sheet may be any one of a uniaxially stretched sheet, a biaxially stretched sheet, or an unstretched sheet. The separation layer may be formed from, for example, a silicone resin.

The carrier layer 212 may include only the substrate sheet described above. In this case, a contact surface of the substrate sheet that is in contact with the substrate layer 214 may be subject to processing for facilitating peeling of the substrate layer 214 from the substrate sheet. The processing on the contact surface of the substrate sheet may be, for example, embossing.

The thickness of the carrier layer 212 may be, for example, between 30 $\mu$m and 400 $\mu$m, inclusive.

The adhesive layer 213 is formed from a synthetic resin. The synthetic resin used to form the adhesive layer 213 may be, for example, a polyurethane resin. The thickness of the adhesive layer 213 may be, for example, between 5 $\mu$m and 25 $\mu$m, inclusive.

The substrate layer 214 is formed from a synthetic resin. The synthetic resin used to form the substrate layer 214 may be, for example, a polyurethane resin. The polyurethane resin may be an aqueous polyurethane. This provides the substrate layer 214 with a relatively high adhesive suitability to the skin. The thickness of the substrate layer 214 may be, for example, between 5 $\mu$m and 30 $\mu$m, inclusive. The substrate layer 214 that is formed from a polyurethane resin and is relatively thin extends well even if the external force applied to the substrate layer 214 for stretching the substrate layer 214 is relatively small. Thus, the substrate layer 214 has relatively high conformity to the shape of the skin, to which the laminate 210L should be adhered, and can achieve relatively high adhesion to the skin.

The substrate layer 214, which has relatively high adhesion to the skin, also has relatively high adhesion to the carrier layer 212, which covers the substrate layer 214. The substrate layer 214, which has relatively high conformity to the shape of the skin, also has relatively high conformity to the carrier layer 212, which covers the substrate layer 214. Thus, when a force is applied to the carrier layer 212 to peel it off from the substrate layer 214, the substrate layer 214 is less likely to be peeled off from the carrier layer 212 and easily deforms following the deformation of the carrier layer 212.

Thus, since the substrate layer 214 is formed from a polyurethane resin, which has relatively high tensile elongation at break, the effect of the adhesive body 210 having the gap 210A is more significant.

The thickness of the substrate layer 214 may be, for example, between 5 $\mu$m and 30 $\mu$m, inclusive.

The tensile elongation at break of the substrate layer 214 may be, for example, greater than or equal to 130%. When the substrate layer 214 has a relatively high tensile elongation at break that is included in the range of greater than or equal to 130%, the effect of the adhesive body 210 having the gap 210A is more significant.

The tensile elongation at break can be obtained in accordance with JIS K 7161-1:2014 (ISO 527-1) "Plastics - Determination of tensile properties - Part 1: General principles" and JIS K 7127:1999 (ISO 527-3) "Plastics - Determination of tensile properties - Part 3: Test conditions for films and sheets." If the measurement object does not have a yield point, the tensile strain at break is measured. If it has a yield point, the nominal tensile strain at break is measured. The tensile elongation at break can be determined using these measurement values.

The tensile strength of the substrate layer 214A at 100% elongation may be, for example, less than or equal to 4 N/cm. The tensile strength at 100% elongation is a value obtained by dividing, by the width of the test piece, the magnitude of the force measured when the strain defined in JIS K 7161 - 1:2014 (ISO 527-1) "Plastics - Determination of tensile properties - Part 1: General principles" reaches a specified value (100%). The tensile strength at 100% elongation (T) (N/cm) can be determined by the following equation.

$$T = F/W$$

In this equation, F is the magnitude of the measured force (N) and W is the width of the test piece (cm).

At least one of the substrate layer 214 and the adhesive layer 213 may contain an active ingredient for skin. That is, only the substrate layer 214 may contain an active ingredient, only the adhesive layer 213 may contain an active ingredient, or both the substrate layer 214 and the adhesive layer 213 may contain an active ingredient. The active ingredient may be, for example, a cosmetic ingredient, a beauty ingredient, or a pharmaceutical ingredient. The active ingredient may be, for example, chitosan or glycerin.

The substrate layer 214 may be formed from synthetic resins other than a polyurethane resin. Examples of synthetic resins other than a polyurethane resin include a polyvinylidene fluoride resin, an ethylene-vinyl acetate copolymer resin, a polypropylene resin, and a polyethylene terephthalate resin.

The grip portion 215 is attached to the carrier layer 212 using adhesive. The grip portion 215 is formed from a synthetic resin. The synthetic resin used to form the grip portion 215 is not particularly limited, if it can be attached the carrier layer 212 with adhesive. For example, it may be a polyethylene terephthalate resin, polypropylene resin, or silicone resin. The thickness of the grip portion 215 may be, for example, between 10 μm and 200 μm, inclusive. The tensile elongation at break of the grip portion 215 is smaller than that of the substrate layer 214.

To use the adhesive body 210, first, the user of the adhesive body 210 detaches the release layer 211 from the adhesive surface 213A of the adhesive layer 213. Next, the user brings the adhesive surface 213A into contact with the skin, thereby adhering the adhesive layer 213 to the skin. Subsequently, the user grips a part of the grip portion 215 projecting outward from the outer shape of the carrier layer 212 to peel off the carrier layer 212 in a direction away from the substrate layer 214.

Since the grip portion 215 overlaps part of the gap 210A, when the user pulls up the grip portion 215 in a direction away from the substrate layer 214, the edge of the curved portion 212A of the carrier layer 212 can be pulled up in a direction away from the substrate layer 214. The separation of the edge of the curved portion 212A from the substrate layer 214 connects the gap 210A, which is a closed space, to the outside of the adhesive body 210. As a result, the carrier layer 212 is peeled off from the substrate layer 214 more easily.

Second Example

A second example of the adhesive body will now be described with reference to Figs. 29 and 30. The second example of the adhesive body is different from the first example of the adhesive body in the number of laminates that are laminated on one release layer. Thus, in the following, the components of the second embodiment that are different from those of the first embodiment will be described, while the components that are common to both the second and first embodiments will be denoted with the same reference numerals as in the first embodiment. Such components will not be described in detail.

Fig. 29 shows a structure of the adhesive body of the second example as seen in the view facing the plane on which the release layer of the adhesive body extends.

As shown in Fig. 29, the adhesive body 220 includes one release layer 221. The release layer 221 has a rectangular shape. In the adhesive body 220 of this example, laminates 210L (see Fig. 30) are located on one release layer 221. The carrier layer 212 is located on each laminate 210L. That is, adhesion units each including the laminate 210L and the carrier layer 212 are located on one release layer 221. For example, in the example shown in Fig. 29, eight adhesion units are located on one release layer 221. The eight adhesion units are arranged on the release layer 221 in two rows, with four adhesion units continuously arranged in each row in the longitudinal direction, in which the long sides of the release layer 221 extend, and the two rows are spaced apart in the lateral direction, in which the short sides of the release layer 221 extend. In the adhesion units arranged in the longitudinal direction, no gap is located between adjacent ones of the adhesion units.

The outer shape of each adhesion unit in the carrier layer 212 has a substantially square shape as seen in a view facing the plane in which the release layer 221 extends. As seen in the view facing the plane in which the release layer 221 extends, the outer shape of the laminate 210L of each adhesion unit has the same shape and the same size as the carrier layer 212. The entire outer shape of the laminate 210L thus corresponds to the entire outer shape of the carrier layer 212.

Each adhesion unit includes the gap 210A. The gap 210A of each adhesion unit extends in the direction in which the long sides of the release layer 221 extend. In the four adhesion units arranged in the longitudinal direction, the gaps 210A of the adhesion units are located on the same straight line extending in the longitudinal direction. In the two adhesion units arranged in the lateral direction, the gap 210A of each adhesion unit is located, in the direction from a first short side to a second short side, along the side of the outer shape of the carrier layer 212 that is closer to the second short side.

As shown in Fig. 30, in the adhesive body 220 of this example, multiple laminates 210L are located on one release layer 221. Each laminate 210L includes the adhesive layer 213 and the substrate layer 214. Each carrier layer 212 is located on a corresponding substrate layer 214.

In the same manner as the release layer 211 of the adhesive body 210 of the first example, the release layer 221 is configured such that the peel strength between the release layer 221 and the adhesive layer 213 is smaller than the peel strength between the carrier layer 212 and the substrate layer 214. The release layer 221 is formed from a synthetic resin. The release layer 221 includes, for example, a substrate sheet and a separation layer. The separation layer is laminated on the substrate sheet. When the release layer 221 includes a substrate sheet and a separation layer, the separation layer is in contact with the adhesive layer 213. The substrate sheet may be formed from, for example, a polyethylene terephthalate resin. The substrate sheet may be any one of a uniaxially stretched sheet, a biaxially stretched sheet, or an unstretched sheet. The separation layer may be formed from, for example, a silicone resin.

The release layer 221 may include only the substrate sheet described above. In this case, a contact surface of the substrate sheet that is in contact with the adhesive surface 213A of the adhesive layer 213 may be subject to processing for facilitating peeling of the adhesive layer 213 from the substrate sheet. The processing on the contact surface of the substrate sheet may be, for example, embossing.

The thickness of the release layer 221 may be, for example, between 12 $\mu$m and 350 $\mu$m, inclusive.

To use the adhesive body 220, first, the user of the adhesive body 220 detaches one of the eight adhesion units from the release layer 221. Next, the user brings the adhesive layer 213 of that adhesive unit into contact with the skin, thereby adhering the adhesive layer 213 to the skin. Subsequently, the user grips a portion of the carrier layer 212 in its outer shape to peel off the carrier layer 212 in a direction away from the substrate layer 214.

In this state, the user can visually observe that the adhesion unit has the gap 210A as seen in a view facing the carrier layer 212 in the adhesion unit adhered to the skin. Thus, the gap 210A also guides the user to peel off the carrier layer 212 from a part relatively near from the gap 210A.

Accordingly, since a part of the carrier layer 212 relatively near from the curved portion 212A can be pulled up in the direction away from the substrate layer 214, the edge of the curved portion 212A can be pulled up in a direction away from the substrate layer 214. The separation of the edge of the curved portion 212A from the substrate layer 214 connects the gap 210A, which is a closed space, to the outside of the adhesive body 210. As a result, the carrier layer 212 is peeled off from the substrate layer 214 more easily.

The adhesive body 220 of this example may include the grip portion 215, in the same manner as the adhesive body 210 of the first example. When the adhesive body 220 includes the grip portion 215, each adhesion unit includes one grip portion 215. In each adhesion unit, the grip portion 215 is attached to the carrier layer 212 so as to overlap part of the gap 210A and protrude outward from the outer shape of the carrier layer 212 as seen in the view facing the plane on which the release layer 221 extends. When the adhesion unit includes the grip portion 215, the user of the adhesive body 220 can grip a part of the grip portion 215 projecting outward from the outer shape of the carrier layer 212 to peel off the carrier layer 212 in a direction away from the substrate layer 214.

Method for Manufacturing Adhesive Body

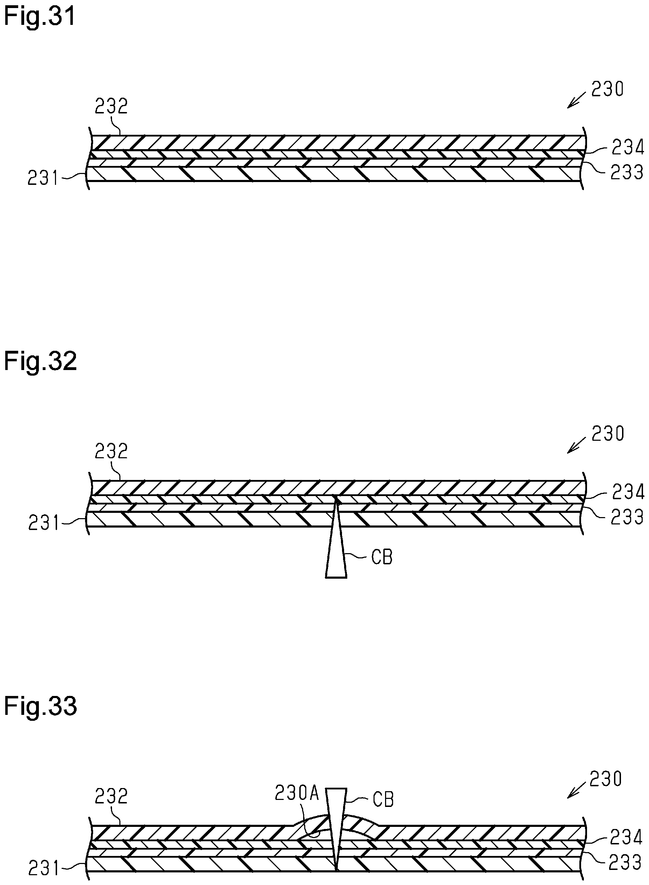

The method for manufacturing the adhesive body 210, 220 will now be described with reference to Figs. 31 to 35.

The method for manufacturing the adhesive body 210, 220 includes forming a precursor of the adhesive body 210, 220 and forming the adhesive body 210, 220. To form the precursor of the adhesive body 210, 220, the precursor of the adhesive body 210, 220 is formed such that the adhesive layer 213 is located between the release layer 211, 221 and the carrier layer 212 and the substrate layer 214 is located between the adhesive layer 213 and the carrier layer 212. To form the adhesive body 210, 220, the adhesive body 210, 220 is formed by cutting the precursor such that the outer shape of the laminate 210L, including the adhesive layer 213 and the substrate layer 214, has a portion that matches the outer shape of the carrier layer 212. Further, forming the adhesive body 210, 220 includes forming the gap 210A, in which the substrate layer 214 is separated from the carrier layer 212 in at least part of the matching portion, by cutting the precursor. The manufacturing method will now be described in more detail with reference to the drawings.

For example, to manufacture the adhesive body 210, 220, first, a synthetic resin sheet that serves as a release layer is prepared. Next, a coating film is formed by applying a coating solution used to form an adhesive layer onto the release layer. Subsequently, the coating film is dried to form the adhesive layer.

The synthetic resin sheet, which serves as the carrier layer, is prepared. Next, a coating solution used to form the substrate layer is applied onto the carrier layer to form a coating film, and then the coating film is dried. Subsequently, the dried coating film is aged at room temperature to form the substrate layer. Thereafter, the adhesive layer is bonded to the substrate layer.

Thus, a precursor 230 of the adhesive body 210, 220 is obtained as shown in Fig. 31. In the precursor 230, an adhesive layer 233 is located between the release layer 231 and the carrier layer 232 and the substrate layer 234 is located between the adhesive layer 233 and the carrier layer 232.

Thereafter, to manufacture the adhesive body 210 of the first example, as shown in Fig. 32, half cutting is performed on the precursor 230 using a cutting blade CB in a direction from the release layer 231 toward the carrier layer 232 so as to extend through the release layer 231, the adhesive layer 233, and the substrate layer 234 and so as not to extend through the carrier layer 232. For example, half cutting is performed on the precursor 230 in a straight shape extending in one direction. As a result, part of the carrier layer 232 is lifted from the substrate layer 34 along the portion that has been cut by the cutting blade CB. This forms a gap 230A at the boundary between the carrier layer 232 and the substrate layer 234.

Next, as shown in Fig. 33, the laminate including the carrier layer 232, the substrate layer 234, the adhesive layer 233, and the release layer 231 is punched using the cutting blade CB into a shape corresponding to the outer shape of the adhesive body 210 in a direction from the carrier layer 232 toward the release layer 231. For example, the carrier layer 212, the substrate layer 234, the adhesive layer 233, and the release layer 231 are punched out such that the outer shape of the carrier layer 232 and the outer shape of the laminate 210L have the same rectangular shape and the same size.

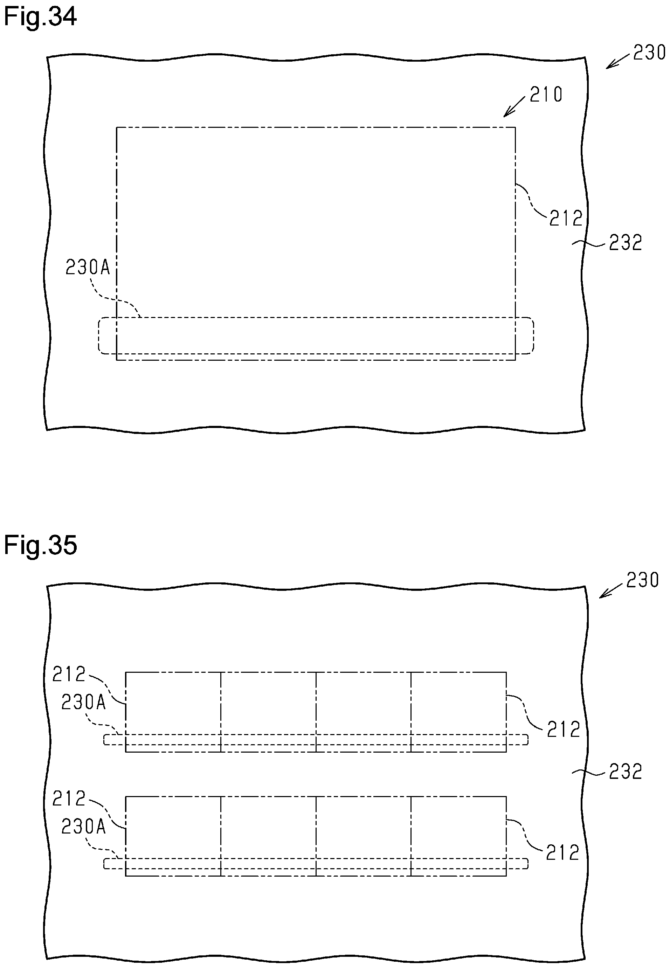

Fig. 34 shows a structure of the precursor 230, as seen in a view facing the carrier layer 232.

As shown in Fig. 34, at the time of punching, part of the precursor 230 is punched along the long dashed double-short dashed line shown in Fig. 34. In the example shown in Fig. 34, the opposite ends of the gap 230A are cut using the cutting blade CB in the direction in which the gap 230A extends. Thus, part of the carrier layer 232 is pressed against part of the substrate layer 234 at the cut portion. This forms the gap 210A, which is a closed space, between the carrier layer 212 and the substrate layer 214.

In this manner, the shapes of the region subjected to half cutting and the region subjected to punching are used to control whether the gap 210A at the boundary between the carrier layer 212 and the substrate layer 214 is an open space, and control the opening position and the size of the opening portion when the gap 210A is open.

In the above-described punching process, half cutting may be performed on the laminate including the carrier layer 232, the substrate layer 234, the adhesive layer 233, and the release layer 231 so as to extend through the carrier layer 232, the substrate layer 234, and the adhesive layer 233 and so as not to extend through the release layer 231.

As a result, in the laminate including the carrier layer 232, the substrate layer 234, and the adhesive layer 233, a second portion surrounding a first portion included in the adhesive body 210 can be separated from the first portion.

Thereafter, in the same manner as when manufacturing the adhesive body 220 of the first example, to manufacture the adhesive body 210 of the second example, half cutting is performed on the precursor 230 in the direction from the release layer 231 toward the carrier layer 232 so as to extend through the release layer 231, the substrate layer 234, and the adhesive layer 233 and so as not to extend through the carrier layer 232. Next, in the direction from the carrier layer 232 toward the release layer 231, half cutting is performed on the precursor 230 in a shape corresponding to the outer shape of the carrier layer 212 so as to extend through the carrier layer 232, the substrate layer 234, and the adhesive layer 233 and so as not to extend through the release layer 231.

As shown in Fig. 35, at the time of half cutting, half cutting is performed on part of the precursor 230 along the long dashed double-short dashed line shown in Fig. 35. Thus, at the boundary between one carrier layer 212 and another carrier layer 212, half cutting is performed in the direction from the carrier layer 232 toward the release layer 231 so as to extend through the carrier layer 232, the substrate layer 234, and the adhesive layer 233 and so as not to extend through the release layer 231. In the example shown in Fig. 35, the opposite ends of the gap 230A are cut using the cutting blade CB in the direction in which the gap 230A extends, and the gap 230A is also cut using the cutting blade CB in the middle of the extension of the gap 230A. This causes part of the carrier layer 232 to be pressed against part of the substrate layer 234 at each cut portion. This forms the gap 210A, which is a closed space, between the carrier layer 212 and the substrate layer 214.

Instead of half cutting, the precursor 230 may be punched. In this case, the release layer is peeled off from the adhesive layer 213 of each laminate formed by punching, and then the laminate including the carrier layer 212, the substrate layer 214, and the adhesive layer 213 is bonded to a new release layer 221.

In addition, to manufacture the adhesive body 210 of the first example and the adhesive body 220 of the second example, the cutting blade used in the half cutting that is a first cutting may be the same as or different from the cutting blade used in the punching or half cutting that is a second cutting.

To perform half cutting on the precursor 230 of the adhesive body 210, 220, a double-edged blade having inclined opposite surfaces is used as the cutting blade CB, and the double-edged blade is inserted and pulled out so as to be orthogonal to a plane on which the release layer 231 extends. Thus, when the double-edged blade is pulled out, the portion of the carrier layer 232 in contact with the double-edged blade is pulled up from the substrate layer 234. This forms the gap 230A between the carrier layer 232 and the substrate layer 234. Thereafter, punching or half cutting is performed in the direction from the carrier layer 232 toward the release layer 231 so as to include at least part of the gap 230A and to be orthogonal to the plane on which the carrier layer 232 extends. Thus, part of the carrier layer 232 adheres to part of the substrate layer 234 at a portion of the gap 230A that is in contact with the cutting blade CB.

Whether the gap 230A is formed between the carrier layer 232 and the substrate layer 234, and, the length of the gap 230A orthogonal to the direction in which the gap 230A extends, can be adjusted by the angle of the double-edged blade and the angle formed by the direction in which the double-edged blade is inserted with respect to the plane on which the release layer 231 extends. The angle of the double-edged blade is formed by two inclined surfaces of the double-edged blade. The smaller the angle between the double-edged blade, the more easily the gap 230A tends to be formed. In addition, the width of the gap 230A tends to increase as the angle of the double-edged blade decreases.

In addition, as the angle formed by the plane on which the release layer 231 extends and the direction in which the double-edged blade is inserted approaches perpendicular, the gap 230A tends to be formed more easily. In addition, as the angle formed by the plane on which the release layer 231 extends and the direction in which the double-edged blade is inserted approaches perpendicular, the width of the gap 230A tends to be greater.

Test Examples

Test Example 3-1

A film (Cerapeel WZ, manufactured by Toray Film Processing Co., Ltd.) including a polyethylene terephthalate film and a separation layer was prepared as a release layer. Further, a urethane-based adhesive (SP-205, manufactured by Toyochem Co., Ltd.) as a main agent and a curing agent (T-501B, manufactured by Toyochem Co., Ltd.) were prepared. After adding the curing agent to the main agent, a mixture of the main agent and the curing agent was stirred to obtain a coating solution for forming the adhesive layer. The coating solution was applied to the release sheet to form a coating film, and then the coating film was dried to obtain an adhesive layer having a thickness of 15 $\mu$m.

A biaxially oriented polypropylene film (FOR-MP, manufactured by Futamura Chemical Co., Ltd.) with one surface being a matte surface was prepared as a carrier layer. Then, an aqueous polyurethane (TAKELAC WS-6021, manufactured by Mitsui Chemicals, Inc.) containing an ether-based polyol was applied to the matte surface to form a coating film, and then the coating film was dried. Next, the dried coating film was aged at room temperature for three days to obtain a substrate layer having a thickness of 15 $\mu$m.

The adhesive layer was bonded to the substrate layer to obtain a precursor of an adhesive body in which the substrate layer was located between the carrier layer and the adhesive layer, and the adhesive layer was located between the substrate layer and the release layer. Then, half cutting was performed on the precursor of the adhesive body in a direction from the release layer toward the carrier layer so as to extend through the substrate layer, the adhesive layer, and the release layer and so as not to extend through the carrier layer. In this state, half cutting was performed on each of the substrate layer, the adhesive layer, and the release layer so as to draw a straight line extending in one direction. In addition, in the half cutting, a double-edged blade having inclined opposite surfaces was used, and the double-edged blade was inserted and pulled out such that the double-edged blade was orthogonal to a plane on which the carrier layer extended. This formed a gap in which the substrate layer was separated from the carrier layer was in a portion of the precursor that was in contact with the double-edged blade.

Next, half cutting was performed on a laminate including the carrier layer, the substrate layer, the adhesive layer, and the release layer in the direction from the carrier layer toward the release layer. In this state, as seen in a view facing the carrier layer, half cutting was performed on the laminate so as to extend through only the carrier layer, the substrate layer, and the adhesive layer and cut the opposite ends of the gap in a direction in which the gap extended. Thus, the adhesive body of test example 3-1 including a gap that was a closed space was obtained.

Test Example 3-2

The adhesive body of test example 3-2 was obtained using the same method as in test example 3-1, except that chitosan (32905-2A, manufactured by Jujo Synthetic Chemical Research Institute Co., Ltd.) was added to the coating solution used to form the adhesive layer in a weight ratio of 200% relative to the main agent in test example 3-1.

Test Example 3-3

The adhesive body of test example 3-3 was obtained using the same method as in test example 3-1, except that glycerin (17116-23, manufactured by Jujo Synthetic Chemical Research Institute Co., Ltd.) was added to the coating solution used to form the adhesive layer in a weight ratio of 40% relative to the main agent in test example 3-1.

Test Example 3-4

The adhesive body of test example 3-4 was obtained in the same manner as in test example 3-1, except that after drying the coating film applied to the matte surface of the carrier layer, the dried coating film was left in an environment at 80°C for two days.

Test Example 3-5

The adhesive body of test example 3-4 was obtained in the same manner as in test example 3-1, except that after drying the coating film applied to the matte surface of the carrier layer, the dried coating film was left in an environment at 80°C for four days.

Test Example 3-6

The adhesive body of test example 3-6 was obtained using the same method as in test example 3-1, except for changing the carrier layer to cellophane tape (CT-15355P, manufactured by Nichiban Co., Ltd.) in test example 3-1.

Evaluation Method

Adhesion Strength

In each test example, a test piece that was 100 mm long and 25 mm wide was cut out from the precursor of the adhesive body. The test piece was cut out from the precursor of the adhesive body such that the test piece did not have a gap. Next, the adhesion strength between the carrier layer and the substrate layer was measured using each test piece. The method in accordance with "180° peel strength" of JIS Z 0237:2009 "Testing methods of pressure-sensitive adhesive tapes and sheets" was used to measure the adhesion strength. Additionally, for measuring the adhesion strength, an Autograph testing machine (Autograph® AGS-X load cell 1kN, manufactured by Shimadzu Corporation) was used, and the test speed was set to 300 mm/min. For each test example, the adhesion strengths of the five test pieces were measured, and then the average value of the adhesion strengths of the five test pieces was calculated. The average value was defined as the adhesion strength in each test example.

Handling and Ease of Peeling

For each test example, half cutting was performed twice on the precursor of the adhesive body, thereby forming five adhesion units each including the carrier layer, the substrate layer, and the adhesive layer on the release layer with 15 cm square. The outer shape of the adhesion unit was set to a rectangular shape in which the long side was 50 mm and the short side was 10 mm. Additionally, a gap with a width of 1 mm in the direction in which the long sides extended was formed along the entire length of one short side of the outer shape of the adhesion unit. It was observed that the gap of each adhesion unit was a closed space.

The adhesive body of each test example was evaluated for the following three items.

(Item 1) Whether the carrier layer was peeled off from the substrate layer when the adhesive layer was peeled off from the release layer

(Item 2) Whether the carrier layer was peeled off from the substrate layer when the adhesion unit was adhered to the skin

(Item 3) Whether the carrier layer could be peeled off from the substrate layer after the adhesion unit was adhered to the skin

Item 1 was evaluated based on the following criteria.

○ When the adhesive layer was peeled off from the release layer, the carrier layer was not peeled off from the substrate layer.

× When the adhesive layer was peeled off from the release layer, part of the carrier layer was peeled off from the substrate layer.

Item 2 was evaluated based on the following criteria.

o When the adhesion unit was adhered to the skin, the carrier layer was not peeled off from the substrate layer.
× When the adhesion unit was adhered to the skin, part of the carrier layer was peeled off from the substrate layer.

Item 3 was evaluated based on the following criteria.

o The carrier layer could be peeled off from the substrate layer after the adhesion unit was adhered to the skin, and no wrinkles occurred in the substrate layer and the adhesive layer from which the carrier layer had been peeled off.
× When the adhesion unit was adhered to the skin and then the substrate layer was peeled off from the carrier layer, part of the adhesive layer was peeled off or wrinkles occurred in the substrate layer and the adhesive layer from which the carrier layer had been peeled off.

For each item, when the evaluation result of one adhesion unit was "×," the evaluation result of the adhesive body including the adhesion unit was "×." That is, only when the evaluation results of all the adhesion units were "o," the evaluation result of each item was set to "o."

Evaluation Results

The results of the evaluation of the adhesive body of each test example were as shown in Table 5 below.

Table 5

|  | Adhesion Strength (N/25mm) | Item 1 | Item 2 | Item 3 |
|---|---|---|---|---|
| Test Example 3-1 | 0.07 | ○ | ○ | ○ |
| Test Example 3-2 | 0.16 | ○ | ○ | ○ |
| Test Example 3-3 | 0.34 | ○ | ○ | ○ |
| Test Example 3-4 | 0.66 | ○ | ○ | ○ |
| Test Example 3-5 | 1.06 | ○ | ○ | ○ |
| Test Example 3-6 | 2.24 | ○ | ○ | × |

As shown in Table 5, it was observed that the adhesion strength of test example 3-1 was 0.07 N/25 mm, the adhesion strength of test example 3-2 was 0.16 N/25 mm, and the adhesion strength of test example 3-3 was 0.34 N/25 mm. It was observed that the adhesion strength of test example 3-4 was 0.66 N/25 mm, the adhesion strength of test example 3-5 was 1.06 N/25 mm, and the adhesion strength of test example 3-6 was 2.24 N/25 mm.
In addition, it was observed that the evaluation result of item 1 and the evaluation result of item 2 were "o" in all of test examples 3-1 to 3-6. These results indicate that when the adhesion strength between the substrate layer and the carrier layer is greater than or equal to 0.07 N/25 mm, it limits situations in which the carrier layer is peeled off from the substrate layer when peeling the adhesive layer from the release layer and the carrier layer is peeled off from the substrate layer when adhering the adhesion unit to the skin.
For test examples 3-2 and 3-3, it is considered that chitosan or glycerin added to the adhesive layer migrated to the substrate layer, causing the swollen substrate layer to bite into the matte surface of the carrier layer, and as a result, the adhesion strength between the carrier layer and the substrate layer was enhanced compared with test example 3-1. In test examples 3-3 and 3-4, it is considered that the substrate layer softened by being left at a temperature of 80°C, which is relatively high, for an extended period, causing the substrate layer to bite into the matte surface of the carrier layer, and as a result, the adhesion strength between the carrier layer and the substrate layer was enhanced compared with test example 3-1.
In addition, it was observed that the evaluation result of item 3 was "o" in test examples 3-1 to 3-5 whereas it was "×" in test example 3-6. These results indicate that the adhesion strength between the substrate layer and the carrier layer is less than or equal to 2N/25 mm, and preferably less than or equal to 1.06 N/25 mm, to enable the carrier layer to be peeled off from the substrate layer of the adhesion unit adhered to the skin.
As described above, the adhesive body and the method for manufacturing the adhesive body of the third embodiment provides the following advantages.

(3-1) The gap 210A, in which the substrate layer 214 is separated from the carrier layer 212, is provided at the portion where the adhesion strength between the substrate layer 214 and the carrier layer 212 is less than or equal to 2 N/25 mm and the outer shape of the laminate 210L matches the outer shape of the carrier layer 212. Thus, the substrate layer 214 having a relatively high tensile elongation at break is easily peeled off from the carrier layer 212.

(3-2) Since the gap 210A is a closed space, the collection of foreign matter on a portion of the substrate layer 214 that is separated from the carrier layer 212 is limited.

(3-3) Since the gap 210A between the carrier layer 212 and the substrate layer 214 extends along the entirety of one side included in the outer shape of the laminate 210L, the substrate layer 214 is more easily peeled off from the carrier layer 212.

(3-4) Since the substrate layer 214 includes a polyurethane resin, which has a relatively high tensile elongation at break, the effect of the adhesive body 210, 220 having the gap 210A is more significant.

(3-5) Since the substrate layer 214 has a relatively high tensile elongation at break that is included in the range of greater than or equal to 130%, the effect of the adhesive body 210, 220 having the gap is more significant.

(3-6) Half cutting is performed on the precursor 230, thereby forming the gap 230A between the carrier layer 232 and the substrate layer 234.

Modifications of Third Embodiment

The above-described third embodiment may be modified as follows.

Grip Portion

The adhesive body 210 of the first example does not have to include the grip portion 215. Even in this case, as described above, the carrier layer 212 can be peeled off from the substrate layer 214 by directly gripping the carrier layer 212.

Gap

When multiple sides of the outer shape of the laminate 210L match the outer shape of the carrier layer 212, the gap 210A may have a shape extending over the sides. For example, when the portion of the outer shape of the laminate 210L that matches the outer shape of the carrier layer 212 has an L-shape, the gap 210A may also have an L-shape. This allows for the formation of the gap 210A when cutting the precursor 230 of the adhesive body 210, 220. Further, for example, multiple sides separated from each other may be included in the portion of the outer shape of the laminate 210L that matches the outer shape of the carrier layer 212. In this case, the adhesive body 210, 220 may include multiple gaps 210A that are separated from each other.

The gap 210A provided in the adhesive body 210, 220 may be connected to the space outside the adhesive body 210, 220. In this case, for example, only part of the edge of the curved portion 12A may be connected to the section outside the adhesive body 210, 220. Alternatively, a gap may be formed by peeling off the carrier layer 212 from the substrate layer 214 at the portion of the outer shape of the laminate 210L that matches the outer shape of the carrier layer 212.

As described above, the combination of the shape of the region subjected to half cutting in the direction from the release layer 231 toward the carrier layer 232 and the shape of the region subjected to punching or half cutting in the direction from the carrier layer 232 toward the release layer 231 is used to control the following. That is, the shape of the region subjected to half cutting and the shape of the region subjected to punching or half cutting are used to control whether part of the gap should be opened and control a portion of the gap that should be opened and the size of that portion.

The gap 210A may be located in the entire portion of the outer shape of the laminate 210L that matches with the outer shape of the carrier layer 212.

Outer Shape

The outer shape of the laminate 210L and the outer shape of the carrier layer 212 may be a polygonal shape other than a quadrangular shape, may be a circular shape, or may be an elliptical shape. In any case, the gap 210A may be provided in part of the portion of the outer shape of the laminate 210L that matches the outer shape of the carrier layer 212. The gap 210A can be formed when cutting the precursor of the adhesive body 210, 220, regardless of the outer shape of the laminate 210L and the outer shape of the carrier layer 212.

Only part of the outer shape of the laminate 210L may be the portion that matches the outer shape of the carrier layer 212. In this case, as seen in the view facing the plane on which the carrier layer 212 extends, the carrier layer 212 is preferably larger than the laminate 210L, and the laminate 210L is preferably located within the carrier layer 212. In this case, the gap 210A may be located at the portion of the outer shape of the laminate 210L that coincides with the outer shape of the carrier layer 212.

Substrate Layer

The synthetic resin used to form the substrate layer 214 may be a resin in which a polyurethane resin and another resin are mixed. Alternatively, the synthetic resin used to form the substrate layer 14 may be a resin other than a polyurethane resin, as described above.

The tensile elongation at break of the substrate layer 214 may be less than 130%. Even in this case, the tensile elongation at break of the substrate layer 214 is higher than those of the release layer 211 and the carrier layer 212. This provides the above-described advantages because of the gap 210A between the carrier layer 212 and the substrate layer 214 and the adhesion strength between the carrier layer 212 and the substrate layer 214.

Fourth Embodiment

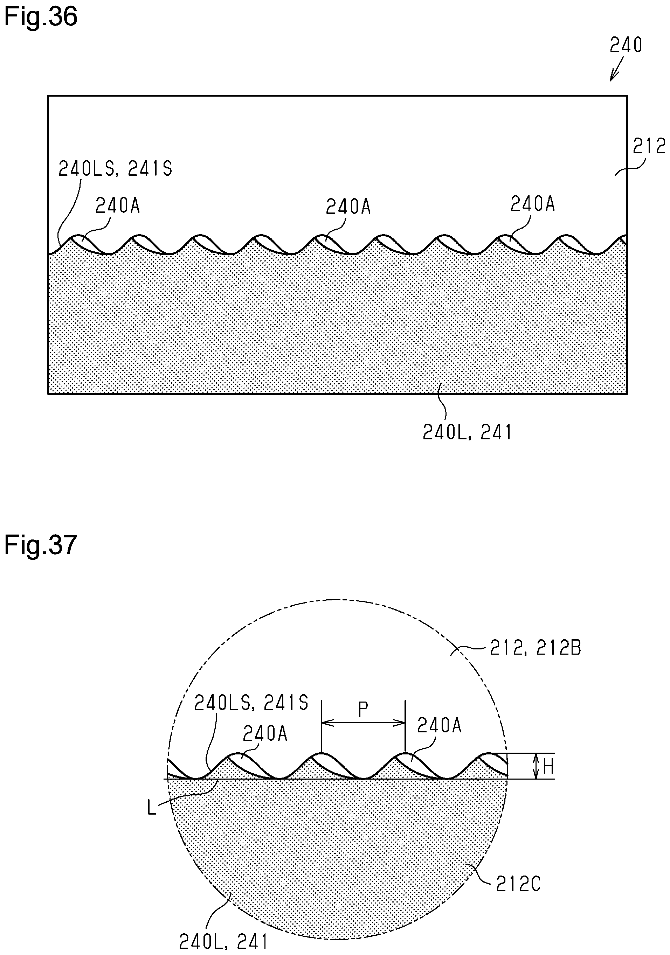

The adhesive body according to a fourth embodiment will now be described with reference to Figs. 36 to 39. The fourth embodiment is different from the third embodiment in the outer shape of the adhesive tape and in the method for forming the gap in the adhesive body. Therefore, in the following, the differences between the third and fourth embodiments will be described in detail, while the components common to the third and fourth embodiments will be represented by the same reference numerals used in the third embodiment and will not be described in detail.

Adhesive Body

The adhesive body will now be described with reference to Figs. 36 and 37. In Figs. 36 and 37, dots are added to the substrate layer is dotted to clearly indicate a region where the substrate layer is located in the carrier layer.

In the same manner as the adhesive body 210 of the third embodiment, an adhesive body 240 of the present embodiment includes the carrier layer 212, a laminate 240L, and a release layer 241. The laminate 240L includes a substrate layer and an adhesive layer. As seen in a view facing the plane on which the carrier layer 212 extends, the outer shape of the laminate 240L includes a portion located within the carrier layer 212. The portion of the outer shape of the laminate 240L that is located within the carrier layer 212 is an inner side 240LS of the laminate 240L.

The inner side 240LS has a wavy shape. The inner side 240LS includes waves. In the example shown in Fig. 36, the waves include waves having the same shape. The inner side 240LS may include waves each having a first shape and waves each having a second shape different from the first shape. The adhesive body 240 has a gap 240A in at least part of the inner side 240LS. Each gap 240A is a space that is open to the space outside the adhesive body 240. In the same manner as the adhesive body 10 of the first embodiment, the adhesive body 240 may include the gap 240A in part of the portion that matches the outer shape of the carrier layer 212 in the outer shape of the laminate 240L. For example, the adhesive body 240 conforms to the outer shape of the carrier layer 212 at the end of the inner side 240LS in the arrangement direction of the waves included in the inner side 240LS. The adhesive body 240 may have the gap 240A at the end of the inner side 240LS in the arrangement direction.

As shown in Fig. 37, the carrier layer 212 includes a first portion 212B in which the laminate 240L is not located and a second portion 212C in which the laminate 240L is located. The inner side 240LS of the laminate 240L is the boundary between the first portion 212B and the second portion 212C as seen in the view facing the plane on which the carrier layer 212 extends. The inner side 240LS of the laminate 240L overlaps an inner side 241S of the release layer 241 having the same wavy shape as the inner side 240LS.

The inner side 240LS of the laminate 240L includes waves arranged in the arrangement direction. Each wave consists of two valleys aligned in the arrangement direction and one peak sandwiched between these valleys. In the example shown in Fig. 37, the waves included in the inner side 240LS each have the same shape. As described above, the waves may include waves each having the first shape and waves each having the second shape, which is different from the first shape. In each wave, the distance between a straight line L passing through two valleys and the peak of the wave is height H of the wave. In the waves, the distance between the peak of each first wave and the peak of a corresponding second wave adjacent thereto in the arrangement direction is a wave cycle P. Height H of each wave may be, for example, in a range of 0.5 mm to 1.5 mm, inclusive. The wave cycle P of the wave may be, for example, in a range of 3 mm to 5 mm, inclusive.

In the example shown in Fig. 37, the adhesive body 240 includes multiple gaps 240A. In the adhesive body 240, one gap 240A is located in each wave on the inner side 240LS of the laminate 240L. As seen in the view facing the plane in which the carrier layer 212 extends, the gap 240A in each wave has a shape including a wave peak and a wave valley. In the example shown in Fig. 37, one gap 240A is located in each wave. Instead, the gap 240A may be located only in at least part of the waves. Further, in the example shown in Fig. 37, the gaps 240A have the same shape. Instead, the gaps 240A may include a gap 240A having the first shape and a gap 240A having the second shape, which is different from the first shape.

Method for Manufacturing Adhesive Body

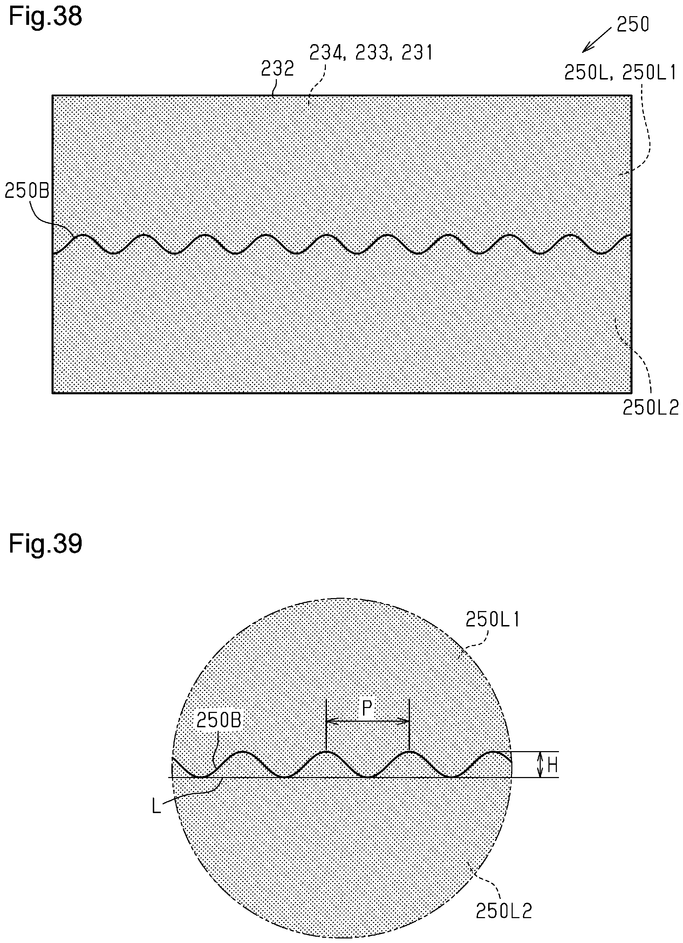

The method for manufacturing the adhesive body will now be described with reference to Figs. 38 and 39. In Figs. 38 and 39, dots are added to the substrate layer is dotted to clearly indicate a region where the substrate layer is located in the carrier layer.

The method for manufacturing the adhesive body of the present embodiment includes forming a precursor and forming a gap, in the same manner as the method for manufacturing the adhesive body of the third embodiment. In the formation of the precursor, the precursor is formed such that an adhesive layer having an adhesive surface that is in contact with a release layer is located between the release layer and a carrier layer and a substrate layer that is in contact with the adhesive layer and the carrier layer is located between the adhesive layer and the carrier layer. The substrate layer has a higher tensile elongation at break than the release layer and the carrier layer. In the formation of the gap, a gap in which the substrate layer is separated from the carrier layer is formed. The gap is located in at least part of the outer shape of a laminate that includes the substrate layer and the carrier layer. The adhesion strength between the substrate layer and the carrier layer is less than or equal to 2 N/25 mm.

In the present embodiment, the formation of the gap includes dividing the laminate into a first portion and a second portion and forming the gap in at least part of a slit. In the dividing of the laminate, a slit that extends through the laminate including the release layer, the adhesive layer, and the substrate layer and does not extend through the carrier layer is formed in a direction from the release layer toward the carrier layer. The slit has a wavy shape as seen in a view facing the plane on which the carrier layer extends. Thus, the laminate is divided into the first portion and the second portion. In the formation of the gap in at least part of the slit, the first portion is peeled off from the second portion and the carrier layer, thereby forming the gap in at least part of the slit. The method for manufacturing the adhesive body in the present embodiment will now be described in more detail with reference to the drawings.

To manufacture the adhesive body, first, a precursor 250 (see Fig. 38) having the same configuration as the precursor 230 described above with reference to Fig. 31 is formed in the same manner as in the method for manufacturing the adhesive body in the third embodiment. That is, after the release layer 231 (see Fig. 38) is prepared, the adhesive layer 233 (see Fig. 38) is formed on one surface of the release layer 231. In addition, after the carrier layer 232 (see Fig. 38) is prepared, the substrate layer 234 (see Fig. 38) is formed on one surface of the carrier layer 232. Subsequently, the adhesive layer 233 is bonded to the substrate layer 234. This provides the precursor 250 such that the adhesive layer 233 is located between the release layer 231 and the carrier layer 232 and the substrate layer 234 is located between the adhesive layer 233 and the carrier layer 232.

Next, as shown in Fig. 38, half cutting is performed on the precursor 250 in the direction from the carrier layer 232 toward the release layer 231 using a cutting blade so as to extend through a laminate 250L, including the carrier layer 232, the substrate layer 234, the adhesive layer 233, and the release layer 231, and so as not to extend through the carrier layer 232. This forms a slit 250B having a wavy shape in the precursor 250. In the arrangement direction in which waves are arranged in the slit 250B, the slit 250B has a length extending over the entire precursor 250. As a result, the slit 250B divides the laminate 250L into a first portion 250L1 and a second portion 250L2.

As shown in Fig. 39, the slit 250B formed in the precursor 250 includes waves arranged in the arrangement direction. Each wave consists of two valleys aligned in the arrangement direction and one peak sandwiched between these valleys. In the example shown in Fig. 39, the waves included in the slit 250B each have the same shape. The waves may include waves each having the first shape and waves each having the second shape, which is different from the first shape. In each wave, the distance between a straight line L passing through two valleys and the peak of the wave is height H of the wave. In the waves, the distance between the peak of each first wave and the peak of a corresponding second wave adjacent thereto in the arrangement direction is a wave cycle P. Height H of each wave may be, for example, in a range of 0.5 mm to 1.5 mm, inclusive. The wave cycle P of the wave may be, for example, in a range of 3 mm to 5 mm, inclusive.

Subsequently, the first portion 250L1 of the laminate 250L is peeled off from the slit 250B, thereby peeling off the first portion 250L1 from the second portion 250L2 and the carrier layer 232. When the first portion 250L1 is separated from the second portion 250L2, a portion of the substrate layer 234 at the slit 250B is pulled in a direction from the second portion 250L2 toward the first portion 250L1, thereby peeling off part of the substrate layer 234 from the carrier layer 232 at the slit 250B. Thus, in at least part of the slit 250B, a gap is formed between the carrier layer 232 and the substrate layer 234. That is, in the second portion 250L2 remaining on the carrier layer 232 within the precursor 250, a gap is formed between the carrier layer 232 and the substrate layer 234 at the boundary with the first portion 250L1. Thus, the adhesive body 240 described above with reference to Fig. 36 is obtained.

Since the inner side 240LS included in the adhesive body 240 is formed by separating the first portion 250L1 from the second portion 250L2 at the slit 250B, the shape of the inner side 240LS is identical with the shape of the slit 250B as seen in the view facing the plane on which the carrier layer 212 extends.

EP 4 501 630 A1

Test Examples

Test examples will now be described with reference to Table 6.

Test Example 4-1

In test example 4-1, a precursor of an adhesive body was obtained by the same method as in test example 3-1. Then, half cutting was performed on the precursor of the adhesive body in a direction from the release layer toward the carrier layer so as to extend through the laminate, including the substrate layer, the adhesive layer, and the release layer, and so as not to extend through the carrier layer. As a result, a slit having a wavy shape and a length extending over the entire precursor in the arrangement direction of waves was formed. Further, in the slit, the height of the wave was set to 1 mm, and the cycle of the wave was set to 4 mm. Next, the laminate was divided into the first portion and the second portion using the slit, and the first portion was separated from the second portion and peeled off from the carrier layer. Consequently, the adhesive body of test example 4-1 was obtained.

Test Example 4-2

In test example 4-2, a precursor of an adhesive body was obtained by the same method as in test example 3-3. Then, half cutting was performed on the precursor of the adhesive body in a direction from the release layer toward the carrier layer so as to extend through the laminate, including the substrate layer, the adhesive layer, and the release layer, and so as not to extend through the carrier layer. As a result, a slit having a wavy shape and a length extending over the entire precursor in the arrangement direction of waves was formed. Further, in the slit, the height of the wave was set to 1 mm, and the cycle of the wave was set to 4 mm. Next, the laminate was divided into the first portion and the second portion using the slit, and the first portion was separated from the second portion and peeled off from the carrier layer. Consequently, the adhesive body of test example 4-2 was obtained.

Evaluation Method

Adhesion Strength

For the adhesive bodies of test examples 4-1 and 4-2, the adhesion strength was evaluated using the same method as that for the adhesive body of the third embodiment.

Handling and Ease of Peeling

For the adhesive bodies of test examples 4-1 and 4-2, handling and ease of peeling were evaluated using the same method as that for the adhesive body of the third embodiment.

Void

For the adhesive bodies of test examples 4-1 and 4-2, whether a gap was located along the inner side having the wavy shape was visually observed.

Evaluation Results

The results of the evaluation of the adhesive bodies of test examples 4-1 and 4-2 were as shown in Table 6 below.

Table 6

|  | Adhesion Strength (N/25mm) | Item 1 | Item 2 | Item 3 | Gap |
|---|---|---|---|---|---|
| Test Example 4-1 | 0.07 | ○ | ○ | ○ | ○ |
| Test Example 4-2 | 0.34 | ○ | ○ | ○ | ○ |

As shown in Table 6, it was observed that the adhesion strength was 0.07 N/25 mm in test example 4-1 and 0.34 N/25 mm in test example 4-2. Further, for all of items 1 to 3, it was observed that the evaluation results of test examples 4-1 and 4-2 were "○." Furthermore, in both of test examples 4-1 and 4-2, it was observed that voids were formed along the inner side of the laminate

As described above, the adhesive body and the method for manufacturing the adhesive body of the fourth embodiment provides the following advantages, in addition to the above-described advantages (301), (3☐4), and (3☐5).

(4-1) Since the substrate layer 244 is peeled off from the carrier layer 242 in at least part of the inner side 240LS, which has a wavy shape, the substrate layer 244 can be easily peeled off as compared with when the substrate layer 244 is bonded to the carrier layer 242 in the entire inner side 240LS.

(4-2) By peeling off the laminate 250L from the first portion 250L1 and the second portion 250L2, a gap can be formed at the boundary between the first portion 250L1 and the second portion 250L2.

Modifications of Fourth Embodiment

The above-described fourth embodiment may be modified as follows.

Grip Portion

The adhesive body 240 may include a grip portion. In this case, the grip portion simply needs to be attached to the first portion 212B of the carrier layer 212 and may have a shape protruding from a portion of the outer edge of the carrier layer 212 included in the first portion 212B.

Outer Shape

The outer shape of the laminate 240L and the outer shape of the carrier layer 212 may be a polygonal shape other than a quadrangular shape, a circular shape, or an elliptical shape.

Substrate Layer

The synthetic resin used to form the substrate layer 244 may be a resin in which a polyurethane resin and another resin are mixed. Alternatively, the synthetic resin used to form the substrate layer 244 may be a resin other than the polyurethane resin, as described above.

The tensile elongation at break of the substrate layer 244 may be less than 130%. Even in this case, the tensile elongation at break of the substrate layer 244 is higher than those of the release layer 241 and the carrier layer 212. This provides the above-described advantages because of the gap 240A between the carrier layer 212 and the substrate layer 244 and the adhesion strength between the carrier layer 212 and the substrate layer 244.

Technical ideas derived from the above-described embodiments, test examples, and modifications will be additionally described.

[Clause 1] An adhesive body, including:

a carrier sheet;
a release sheet; and
an adhesive tape located between the carrier sheet and the release sheet, where
the adhesive tape includes:

a substrate that is in contact with the carrier sheet and has a higher tensile elongation at break than the carrier sheet and the release sheet; and
an adhesive layer that is in contact with the release sheet, the adhesive layer being configured to be adhered to a living body surface after the release sheet is peeled off from the adhesive layer,

the carrier sheet has a larger area than the adhesive tape, and the adhesive tape is located within the carrier sheet as seen in a view facing a plane on which the carrier sheet extends, and
the carrier sheet includes a slit extending toward the adhesive tape from a portion of an outer edge of the carrier sheet that does not overlap the adhesive tape and having a length that does not reach an outer edge of the adhesive tape as seen in the view facing the plane on which the carrier sheet extends, and
in a tear test using a right angled tear method in accordance with JIS K 7128 3:1998,

a displacement amount (mm) of the adhesive tape at break is 3.0 times or more than a displacement amount (mm) of the carrier sheet at break, and
an integral value of a tear resistance (N/mm) of the carrier sheet until break is less than or equal to 1000 N.

In the adhesive body according to clause 1, the displacement amount of the adhesive tape at break is 3.0 times or more than the displacement amount of the carrier sheet at break. Thus, even when a force stretching the carrier sheet acts on the carrier sheet so that it is stretched to such an extent that the carrier sheet breaks, the adhesive tape is less likely to break. Further, the integral value of the tear resistance of the carrier sheet until break is less than or equal to 1000 N. Thus, an excessively large force is not required until break after the application of load to the carrier sheet starts. Accordingly, the carrier sheet can be easily peeled off from the adhesive tape by breaking the carrier sheet.

**Claims**

1. An adhesive body, comprising:

   a carrier sheet;
   a release sheet; and
   an adhesive tape located between the carrier sheet and the release sheet, wherein

   the adhesive tape includes:

   a substrate that is in contact with the carrier sheet and has a higher tensile elongation at break than the carrier sheet and the release sheet; and
   an adhesive layer that is in contact with the release sheet, the adhesive layer being configured to be adhered to a living body surface after the release sheet is peeled off from the adhesive layer,

   the carrier sheet has a larger area than the adhesive tape, and the adhesive tape is located within the carrier sheet as seen in a view facing a plane on which the carrier sheet extends, and
   the carrier sheet includes a slit extending toward the adhesive tape from a portion of an outer edge of the carrier sheet that does not overlap the adhesive tape and having a length that does not reach an outer edge of the adhesive tape as seen in the view facing the plane on which the carrier sheet extends.

2. The adhesive body according to claim 1, wherein
   a trouser tear resistance of the carrier sheet in accordance with JIS K 7128-1:1998 is less than or equal to 10 N/mm.

3. The adhesive body according to claim 1 or 2, wherein, in the carrier sheet,

   the carrier sheet has a rectangular shape with a width of 15 mm and a length of 150 mm,
   a circumference of a loop formed by the carrier sheet is 85 mm, and
   a loop stiffness flexural rigidity when a compression distance is set to 20 mm is less than or equal to 50 mN/15 mm.

4. The adhesive body according to any one of claims 1 to 3, wherein

   the substrate includes a polyurethane resin, and
   a tensile strength at break of the substrate in accordance with JIS K 7161-1:2014 is greater than or equal to 10 MPa.

5. The adhesive body according to any one of claims 1 to 4, wherein
   a 180° peel strength between the carrier sheet and the substrate in accordance with JIS Z 0237:2009 is less than or equal to 550 mN/25 mm.

6. The adhesive body according to any one of claims 1 to 5, wherein
   a peel strength of the adhesive layer in accordance with JIS Z 0237:2009 is greater than or equal to 1 N/25 mm.

7. The adhesive body according to any one of claims 1 to 6, wherein

   the slit is a first slit,
   the carrier sheet further includes a second slit extending toward the adhesive tape from the portion of the outer edge of the carrier sheet that does not overlap the adhesive tape and having the length that does not reach the outer edge of the adhesive tape as seen in the view facing the plane on which the carrier sheet extends, and
   the first slit and the second slit face each other with the adhesive tape located in between as seen in the view facing the plane on which the carrier sheet extends.

8. The adhesive body according to any one of claims 1 to 6, wherein

the slit is a first slit,
the carrier sheet further includes a second slit extending from the portion of the outer edge of the carrier sheet that does not overlap the adhesive tape toward the adhesive tape and having the length that does not reach the outer edge of the adhesive tape as seen in the view facing the plane on which the carrier sheet extends,
as seen in the view facing the plane on which the carrier sheet extends, the first slit and the second slit are spaced apart from each other in a first direction and each of the slits extends in a second direction that intersects the first direction, and
the first slit and the second slit define a grip portion in the carrier sheet, the grip portion being located between the first slit and the second slit and extending in the second direction.

9. The adhesive body according to any one of claims 1 to 8, wherein
the adhesive tape has a quadrangular shape or a circular shape as seen in the view facing the plane on which the carrier sheet extends.

10. The adhesive body according to any one of claims 1 to 8, wherein

the adhesive tape has a trapezoidal shape as seen in the view facing the plane on which the carrier sheet extends,
a length of the adhesive tape in a width direction monotonically decreases from a first end toward a second end of the adhesive tape in an extending direction that is orthogonal to the width direction,
a length of the carrier sheet in the width direction monotonically decreases from the first end toward the second end of the carrier sheet in the extending direction, and
the slit is positioned closer to the second end of the carrier sheet than to a center of the carrier sheet in the extending direction of the carrier sheet.

11. An adhesive body, comprising:

a carrier sheet;
a release sheet; and
an adhesive tape located between the carrier sheet and the release sheet,

wherein

the adhesive tape includes:

a substrate that is in contact with the carrier sheet and has a higher tensile elongation at break than the carrier sheet and the release sheet; and
an adhesive layer that is in contact with the release sheet, the adhesive layer being configured to be adhered to a living body surface after the release sheet is peeled off from the adhesive layer,

the carrier sheet has a larger area than the adhesive tape, and the adhesive tape is located within the carrier sheet as seen in a view facing a plane on which the carrier sheet extends,
the carrier sheet includes a slit extending toward the adhesive tape from a portion of an outer edge of the carrier sheet that does not overlap the adhesive tape and having a length that does not reach an outer edge of the adhesive tape as seen in the view facing the plane on which the carrier sheet extends, and
in a tear test using a right angled tear method in accordance with JIS K 7128 3:1998,

a displacement amount (mm) of the adhesive tape at break is 3.0 times or more than a displacement amount (mm) of the carrier sheet at break, and
an integral value of a tear load applied to the carrier sheet until break is less than or equal to 40 N·mm.

12. The adhesive body according to claim 11, wherein

a maximum tear load (N) of the carrier sheet is less than or equal to 20 N, and
the displacement amount (mm) of the carrier sheet at break is less than or equal to 12 mm.

13. The adhesive body according to claim 11, wherein

in the tear test using the right angled tear method, an integral value of a tear resistance (N/mm) of the carrier sheet until break is less than or equal to 1000 N.

14. The adhesive body according to claim 13, wherein

the tear resistance (N/mm) of the carrier sheet is less than or equal to 376 N/mm, and
the displacement amount (mm) of the carrier sheet at break is less than or equal to 12 mm.

15. The adhesive body according to any one of claims 11 to 14, wherein
in the tear test using the right angled tear method, the displacement amount (mm) of the carrier sheet when the tear load applied to the carrier sheet is 5 N is greater than or equal to 1.5 mm.

16. The adhesive body according to any one of claims 11 to 14, wherein
in the tear test using the right angled tear method, the tear load applied to the carrier sheet when the displacement amount of the carrier sheet is 1 mm is less than or equal to 3.5 N.

17. The adhesive body according to any one of claims 11 to 14, wherein

the substrate includes a polyurethane resin, and
the carrier sheet includes any one of a polypropylene resin, a polyethylene terephthalate resin, and a polyvinylidene fluoride resin.

18. An adhesive body, comprising:

a release layer;
a carrier layer;
an adhesive layer located between the release layer and the carrier layer, the adhesive layer having an adhesive surface that is in contact with the release layer; and
a substrate layer that is located between the adhesive layer and the carrier layer, is in contact with the adhesive layer and the carrier layer, and has a higher tensile elongation at break than the release layer and the carrier layer, wherein
the adhesive surface from which the release layer has been peeled off is configured to be adhered to skin,
the adhesive body further comprises a gap in which the substrate layer is separated from the carrier layer, the gap being located in at least part of an outer shape of a laminate that includes the adhesive layer and the substrate layer, and
an adhesion strength between the substrate layer and the carrier layer is less than or equal to 2 N/25 mm.

19. The adhesive body according to claim 18, wherein

the outer shape of the laminate includes a portion that matches an outer shape of the carrier layer, and
the gap is included in at least part of the matching portion.

20. The adhesive body according to claim 19, wherein
the gap is a closed space.

21. The adhesive body according to claim 19, wherein

the outer shape of the laminate includes sides,
one of the sides is included in the matching portion, and
the gap has a length extending over the entirety of the one side.

22. The adhesive body according to claim 18, wherein

the outer shape of the laminate includes a portion located within the carrier layer as seen in the view facing the plane on which the carrier layer extends,
the portion located within the carrier layer has a wavy shape, and
at least part of the portion located within the carrier layer includes the gap.

23. The adhesive body according to any one of claims 18 to 22, wherein
the substrate layer includes a polyurethane resin.

24. The adhesive body according to any one of claims 18 to 22, wherein
a tensile elongation at break of the substrate layer is greater than or equal to 130%.

25. A method for manufacturing an adhesive body, the method comprising:

forming a precursor such that an adhesive layer having an adhesive surface that is in contact with a release layer is located between the release layer and a carrier layer and such that a substrate layer that is in contact with the adhesive layer and the carrier layer is located between the adhesive layer and the carrier layer, the substrate layer having a higher tensile elongation at break than the release layer and the carrier layer; and
forming a gap in which the substrate layer is separated from the carrier layer, the gap being located in at least part of an outer shape of a laminate that includes the substrate layer and the carrier layer, wherein
an adhesion strength between the substrate layer and the carrier layer is less than or equal to 2 N/25 mm.

26. The method for manufacturing the adhesive body according to claim 25, wherein
the forming the gap includes performing half cutting on the precursor so as to extend through the release layer, the adhesive layer, and the substrate layer and so as not to extend through the carrier layer in a direction from the release layer toward the carrier layer, thereby forming a gap in a boundary between the carrier layer and the substrate layer.

27. The method for manufacturing the adhesive body according to claim 25, wherein
the forming the gap includes:

forming a slit that extends through a laminate including the release layer, the adhesive layer, and the substrate layer and does not extend through the carrier layer in a direction from the release layer toward the carrier layer, thereby dividing the laminate into a first portion and a second portion, wherein the slit has a wavy shape as seen in a view facing a plane on which the carrier layer extends; and
peeling off the first portion from the second portion and the carrier layer, thereby forming the gap in at least part of the slit.

Fig.1

Fig.2

Fig.3

11    13 (12)    10

11A2    11A1

11E

Fig.4

11    10

13    11A1
D
11C
11A2
L

Fig.5

Fig.6

Fig.7

Fig.8

11

13

10

11A1

11C

11A2

11D

Fig.9

10

11

11A

13 (12)

Fig.10

13P2 — 11

11A

11B

13P1 — 13

10

Fig.11

11 — 11A — 10

13, 13E1, 11E1 — 13E2, 11E2

Fig.12

S

11

11A

13, 13E1, 11E1

10

13E2, 11E2

Fig.13

S

11A

11

13

10

13E2, 11E2

13E1, 11E1

Fig.14

S

11A

11

13

10

13E2, 11E2

13E1, 11E1

Fig.15

Fig.16

Fig.17

## Fig.18

## Fig.19

## Fig.20

## Fig.21

Fig.22

Fig.23

## Fig.24

## Fig.25

Fig.26

Fig.27

**Fig.28**

**Fig.29**

**Fig.30**

Fig.31

230

232

234
233

231

Fig.32

230

232

234
233

231

CB

Fig.33

230

232

230A    CB

234
233

231

Fig.34

Fig.35

## Fig.36

240

240LS, 241S

240A 240A 240A

212

240L, 241

## Fig.37

212, 212B

240LS, 241S

P

240A 240A

H

L

212C

240L, 241

Fig.38

250
250L, 250L1

232   234, 233, 231

250B

250L2

Fig.39

250L1

250B

P

H

L

250L2

## INTERNATIONAL SEARCH REPORT

| | International application No. |
|---|---|
| | **PCT/JP2023/013695** |

| A. | CLASSIFICATION OF SUBJECT MATTER |
|---|---|

*B32B 27/00*(2006.01)i; *A61K 9/70*(2006.01)i; *A61F 13/02*(2006.01)i; *C09J 7/38*(2018.01)i; *B32B 7/06*(2019.01)i
FI: A61F13/02 380; C09J7/38; B32B27/00 M; B32B7/06; A61F13/02 310Z; A61F13/02 310D; A61K9/70

According to International Patent Classification (IPC) or to both national classification and IPC

| B. | FIELDS SEARCHED |
|---|---|

Minimum documentation searched (classification system followed by classification symbols)

B32B27/00; A61K9/70; A61F13/02; C09J7/38; B32B7/06

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2010-29242 A (ALCARE CO., LTD.) 12 February 2010 (2010-02-12) | 1, 18-25 |
| | paragraphs [0018]-[0049], fig. 1-17 | |
| Y | | 2-17 |
| A | | 26-27 |
| Y | JP 2011-5028 A (KYOWA LTD.) 13 January 2011 (2011-01-13) | 2-17 |
| | paragraph [0022] | |
| Y | JP 2020-92737 A (TOPPAN PRINTING CO., LTD.) 18 June 2020 (2020-06-18) | 2-17 |
| | paragraph [0062] | |
| X | JP 5384730 B2 (NICHIBAN CO., LTD.) 08 January 2014 (2014-01-08) | 18-26 |
| | paragraphs [0082]-[0097], fig. 1-5 | |
| A | | 27 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **08 June 2023** | **20 June 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/013695**

| Patent document cited in search report | | | Publication date (day/month/year) | Patent family member(s) | | | Publication date (day/month/year) |
|---|---|---|---|---|---|---|---|
| JP | 2010-29242 | A | 12 February 2010 | (Family: none) | | | |
| JP | 2011-5028 | A | 13 January 2011 | (Family: none) | | | |
| JP | 2020-92737 | A | 18 June 2020 | US<br>paragraph [0369]<br>WO<br>EP<br>CN | 2021/0186652<br><br>2020/059861<br>3854314<br>112996438 | A1<br><br>A1<br>A1<br>A | |
| JP | 5384730 | B2 | 08 January 2014 | US<br>paragraphs [0121]-[0135], fig.<br>1-5<br>WO<br>EP<br>CN<br>KR | 2013/0152944<br><br><br>2012/029127<br>2612630<br>103079506<br>10-2013-0137614 | A1<br><br><br>A1<br>A1<br>A<br>A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6706398 B

**Non-patent literature cited in the description**

- Plastics - Determination of tensile properties - Part 1: General principles. *JIS K 7161-1:2014 (ISO 527-1)*
- Plastics - Determination of tensile properties - Part 3: Test conditions for films and sheets. *JIS K 7127:1999 (ISO 527-3)*
- Plastics - Determination of tensile properties - Part 1: General principles. *JIS K 7161 - 1:2014 (ISO 527-1)*
- Plastics - Film and sheeting - Determination of tear resistance-Part 1: Trouser tear method. *JIS K 7128-1:1998*
- Testing methods of pressure-sensitive adhesive tapes and sheets. *JIS Z 0237:2009*
- Plastics - Film and sheeting - Determination of tear resistance - Part 3: Right angled tear method. *JIS K 7128-3:1998*